# FASCICULE DE BREVET EUROPEEN

(11) **EP 1 172 077 B1**
(45) Date de publication et mention de la délivrance du brevet: **21.05.2003**
(21) Numéro de dépôt: 01401832.9
(22) Date de dépôt: 09.07.2001
(51) Int. Cl.: A61K 7/00, B01F 17/00

(54) **Nanoémulsion contentant des polymères non ioniques**
Nanoemulsion enthaltend nicht-ionische Polymere
Nanoemulsion containing nonionic polymers

(30) Priorité: 13.07.2000 FR 0009222
(43) Date de publication de la demande: 16.01.2002
(73) Titulaire: L'OREAL, 75008 Paris (FR)
(72) Inventeur: L'Alloret, Florence, 75013 Paris (FR); Aubrun-Sonneville Odile, 92160 Antony (FR); Simonnet, Jean-Thierry, 75011 Paris (FR)
(74) Mandataire: Rasson, Catherine

(56) Documents cités:
- EP-A- 0 696 452
- EP-A- 0 728 460
- EP-A- 0 968 704
- WO-A-00/61083
- DE-A- 19 641 672
- DE-A- 19 816 662
- FR-A- 2 804 015

## Description

La présente invention a trait à une nanoémulsion, comprenant au moins un lipide amphiphile non ionique et/ou anionique et au moins un polymère hydrosoluble neutre, ainsi qu'à l'utilisation de ladite nanoémulsion en application topique notamment dans les domaines cosmétique et dermatologique, et dans les domaines pharmaceutique et/ou ophtalmologique.

Les émulsions huile-dans-eau (H/E) sont bien connues dans le domaine de la cosmétique et de la dermatologie, notamment pour la préparation de produits cosmétiques tels que des laits, des crèmes, des toniques, des sérums, des eaux de toilette.

Les nanoémulsions sont des émulsions H/E caractérisées par une taille des globules huileux inférieure à 100 nm, les globules huileux étant stabilisés par une couronne de lipides amphiphiles pouvant éventuellement former une phase cristal liquide de type lamellaire, situés à l'interface huile/phase aqueuse. La transparence de ces émulsions provient de la petite taille des globules huileux, petite taille obtenue grâce à l'utilisation d'une énergie mécanique et notamment d'un homogénéisateur haute pression. Les nanoémulsions sont à différencier des microémulsions de par leur structure. En effet, les microémulsions sont des dispersions thermodynamiquement stables constituées de micelles de lipide(s) amphiphile(s) gonflées par de l'huile. De plus, les microémulsions ne nécessitent pas d'énergie mécanique importante pour être réalisées; elles se forment spontanément par simple mise en contact des constituants. Les inconvénients majeurs des microémulsions sont liés à leur forte proportion en tensioactifs, conduisant à des intolérances et entraînant un toucher collant lors de l'application sur la peau. Par ailleurs, leur domaine de formulation est en général très étroit et leur stabilité en température très limitée.

Les nanoémulsions comprennent un ou plusieurs lipide(s) amphiphile(s). Par lipide amphiphile, on entend ici toutes molécules ayant une structure bipolaire, c'est-à-dire comportant au moins une partie hydrophobe et au moins une partie hydrophile et ayant la propriété de réduire la tension superficielle de l'eau (γ< 55mN/m) et de réduire la tension interfaciale entre l'eau et une phase huileuse. Les synonymes de lipide amphiphile sont par exemple: tensioactif, agent de surface, émulsionnant

Les documents EP-A-728 460 et EP-A-780 114 décrivent des nanoémulsions à base de lipides amphiphiles non ioniques liquides ou de tensioactifs siliconés. Des nanoémulsions sont également décrites dans les documents FR-A-2,787,026, FR-A-2,787,027, FR-A-2,787,325, FR-A-2,787,326, FR-A-2,787,703, FR-A-2,787,728.

Pour que les nanoémulsions telles que décrites dans ces documents puissent être utilisées comme laits ou crèmes, et notamment dans le domaine du soin, il faut les rendre plus épaisses et donc augmenter leur viscosité. existe deux moyens pour augmenter la viscosité d'une nanoémulsion. Le premier moyen consiste à augmenter la fraction de la phase huileuse dispersée. En effet, à partir de 22 % en poids de phase huileuse par rapport au poids total de la composition, on constate généralement que la viscosité augmente en fonction du taux d'huile. Cette méthode, décrite dans les demandes citées ci-dessus, permet d'obtenir des compositions épaisses, transparentes et stables. Toutefois, la contrainte d'une telle méthode d'épaississement des nanoémulsions est l'obligation d'avoir un taux d'huile élevé, ce qui n'est pas toujours souhaité car les formules obtenues sont plus riches (taux élevé de phase grasse) et la gamme de viscosité est plus étroite.

Le second moyen pour augmenter la viscosité d'une nanoémulsion consiste à ajouter à la nanoémulsion un polymère hydrosoluble qui, par gélification de la phase continue aqueuse, va augmenter la viscosité de l'ensemble, et ce, même avec de faibles taux d'huile. Dans les demandes citées ci-dessus, il est envisagé l'adjonction de polymères hydrosolubles tels que l'hydroxypropylcellulose, les dérivés d'algues, les gommes naturelles et les polymères synthétiques comme les polymères et copolymères d'acides carboxyvinyliques, par exemple les Carbopols qui sont des polymères anioniques. Malheureusement, l'addition de tels polymères affecte la transparence des produits obtenus ; ou alors pour garder la transparence, il faut mettre peu de polymère, ce qui limite leur effet d'épaississement de la composition.

Ainsi, il subsiste le besoin d'un système épaississant permettant d'épaissir de manière convenable une composition sous forme de nanoémulsion huile-dans-eau, sans influer sur les propriétés cosmétiques desdites compositions, notamment sans influer sur le caractère transparent de la nanoémulsion, et ce quel que soit le taux d'huile que l'on souhaite utiliser.

La demanderesse a découvert, de façon inattendue, que l'on pouvait épaissir les nanoémulsions, avec certains polymères non ioniques (neutres) hydrosolubles. Ces polymères sont hydrosolubles et totalement exempts de chaîne hydrophobe.

La présente invention a pour objet une nanoémulsion huile-dans-eau comportant une phase huileuse dispersée dans une phase aqueuse, dont les globules d'huile ont une taille moyenne en nombre inférieure à 100 nm, caractérisée en ce qu'elle comprend (i) au moins un lipide amphiphile choisi parmi les lipides amphiphiles non ioniques et les lipides amphiphiles anioniques, (ii) au moins un polymère non ionique hydrosoluble totalement exempt de chaîne hydrophobe choisi parmi les homopolymères et copolymères d'oxyde d'éthylène ayant une masse molaire égale ou supérieure à 10 000 g/mole ; les alcools polyvinyliques ; les homopolymères et copolymères de vinylpyrrolidone ; les homopolymères et copolymères de vinylcaprolactame; les homopolymères et copolymères de polyvinylméthyléther ; les homopolymères et copolymères acryliques neutres ; les alkyl-C₁-C₂-celluloses et leurs dérivés ; les alkyl-C₁-C₃-guar ou hydroxyalkyl-C₁-C₃-guar, et (iii) au moins un lipide amphiphile ionique additionnel choisi parmi :
- les sels alcalins du dicétyl- et du dimyristylphosphate ;
- les sels alcalins du cholestérol sulfate ;
- les sels alcalins du cholestérol phosphate ;
- les lipoaminoacides et leurs sels ;
- les sels de sodium de l'acide phosphatidique ;
- les phospholipides ;
- les dérivés alkylsulfoniques ;
- les sels d'ammonium quatemaire,
- les amines grasses ;
- et leurs mélanges ;
et en ce que le rapport pondéral de la quantité de phase huileuse sur la quantité de lipide amphiphile va de 2 à 10, mieux de 2 à 6 et encore mieux de 3 à 6.

Ces polymères totalement compatibles avec les nanoémulsions permettent de les épaissir ou de les gélifier dans une large gamme de viscosités, même à faible taux d'huile, tout en maintenant une bonne transparence des nanoémulsions. Ils permettent d'augmenter la viscosité de la nanoémulsion d'au moins un facteur 5, pour une concentration en polymère égale à 1 % en poids. Ils permettent d'obtenir des compositions transparentes et stables, constituant des laits ou des crèmes. On entend par « lait » ou « crème » des compositions ayant une viscosité allant de 1 à 200 Poises (soit 0,1 Pa.s à 20 Pa.s) mesurée à 25°C avec Rheomat RM 180 au mobile 3, 4 ou 5 (selon la gamme de viscosité), à 200 s⁻¹.

Un autre objet de l'invention est un procédé pour épaissir une nanoémulsion huile-dans-eau ayant des globules d'huile dont la taille moyenne en nombre est inférieure à 100 nm, consistant à ajouter à ladite nanoémulsion au moins un polymère non ionique hydrosoluble, totalement exempt de chaîne hydrophobe, choisi parmi les homopolymères et copolymères d'oxyde d'éthylène ayant' une masse molaire égale ou supérieure à 10 000 g/mole ; les alcools polyvinyliques ; les homopolymères et copolymères de vinylpyrrolidone ; les homopolymères et copolymères de vinylcaprolactame ; les homopolymères et copolymères de polyvinylméthyléther ; les homopolymères et copolymères acryliques neutres ; les alkyl-C₁-C₂-celluloses et leurs dérivés ; les alkyl-C₁-C₃-guar ou hydroxyalkyl-C₁-C₃-guar, la présence du polymère augmentant la viscosité de la nanoémulsion d'au moins un facteur 5 pour une concentration en polymère égale à 1 % en poids.

Les nanoémulsions selon l'invention ont généralement un aspect transparent à bleuté. Leur transparence se mesure par un coefficient de transmittance à 600 nm allant de 10 à 90 % ou bien par une turbidité. La turbidité des compositions de l'invention va de 60 à 400 NTU et de préférence de 70 à 300 NTU, turbidité mesurée au turbidimètre portatif HACH - Modèle 2100 P à environ 25°C.

Les globules d'huile des nanoémulsions de l'invention ont une taille moyenne en nombre, inférieure à 100 nm et de préférence allant de 20 à 80 nm et plus préférentiellement de 40 à 60 nm. La diminution de la taille des globules permet de favoriser la pénétration des actifs dans les couches superficielles de la peau (effet véhicule).

Les nanoémulsions conformes à l'invention sont préparées de préférence à des températures allant de 4 à 45°C et sont ainsi compatibles avec des actifs thermosensibles.

Les polymères utilisés selon la présente invention sont hydrosolubles, c'est-à-dire solubles dans l'eau, et non ioniques c'est-à-dire neutres.

Les polymères neutres hydrosolubles utilisés selon l'invention sont choisis parmi les polymères décrits ci-dessous et leurs mélanges.
A) les homopolymères et copolymères d'oxyde d'éthylène, ayant une masse molaire égale ou supérieure à 10 000 g/mole et de préférence allant 10 000 g/mole à 10 000 000 g/mole. Il peuvent être choisis parmi :
   (1) les poly-oxydes d'éthylène ayant la formule (I) suivante :

      R-(CH₂-CH₂-O)ₙ-R' (I)

      dans laquelle R est choisi parmi les groupes hydroxyle (OH), méthoxy (OCH₃) et amine (NH₂), R' est un groupe méthyl (CH₃) ou un hydrogène, et n est un nombre allant de 220 à 230 000.
   (2) les copolymères d'oxyde d'éthylène et d'un ou plusieurs monomères oxyalkylénés ayant la formule (II) suivante :

      -(CHR-CHR'-O)- (II)
   dans laquelle R et R' indépendamment l'un de l'autre sont un hydrogène ou un groupe alkyle comportant de 1 à 7 atomes de carbone, l'un au moins de R ou R' étant un groupe alkyle.
   Parmi les homopolymères et copolymères d'oxyde d'éthylène, on peut citer notamment les produits commercialisés sous les dénominations Polyox Coagulant (masse molaire d'environ 5.10⁶ g/mole) (nom CTFA : PEG-115M) et Polyox WSR N-60K CG (nom CTFA : PEG-45M) (masse molaire d'environ 2.10⁶ g/mole) par la société Amerchol, ainsi que le produit commercialisé sous la dénomination Carbowax 20M (nom CTFA: PEG-350) (masse molaire d'environ 2.10⁷ g/mole) par la société Union Carbide.
B) les alcools polyvinyliques, notamment ceux ayant une masse molaire moyenne allant de 10 000 g/mole à 500 000 g/mole. Ce sont des composés représentés par la formule (III) suivante : 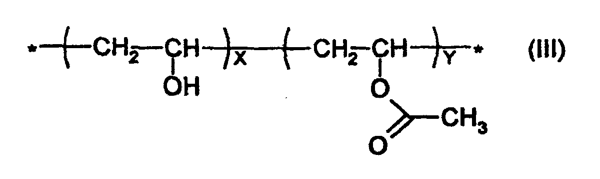 dans laquelle x est un nombre moyen exprimé en pourcentage allant de 70 à 100 ; y est un nombre moyen égal à 100 - x.
   On peut citer par exemple les produits commercialisés sous les dénominations Airvols 103, 350, 203, 540, 714 et 603 par la société Air Products.
C) les homopolymères et copolymères de vinylpyrrolidone, notamment ceux ayant une masse molaire moyenne allant de 10 000 g/mole à 1 000 000 g/mole. Ils peuvent être choisis parmi :
   1) les polyvinylpyrrolidones ayant la formule (IV) suivante : 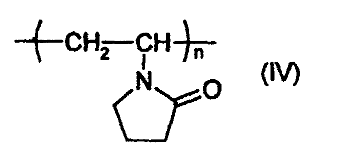 On peut citer par exemple les produits commercialisés sous les dénominations Polyclar V15 (masse molaire d'environ 8 000 g/mole), V30 (masse molaire d'environ 50 000 g/mole), V60 (masse molaire d'environ 400 000 g/mole), V90 (masse molaire d'environ 1 000 000 g/mole) et V120 (masse molaire d'environ 2 500 000 g/mole) par la société ISP.
   2) les copolymères de vinylpyrrolidone tels que :
      (a) les copolymères de vinylpyrrolidone et d'acétate de vinyle, notamment le copolymère contenant 30% d'acétate de vinyle, commercialisé sous la dénomination PVP-VA 735 par la société ISP ;
      (b) les copolymères de vinylpyrrolidone et de dérivés de vinylpyrrolidone à greffons butène comme le copolymère contenant 10 % de vinylpyrrolidone à greffons butène, commercialisé sous la dénomination Ganex (ou Antaron) P904 par la société ISP ;
      (c) les copolymères de vinylpyrrolidone et d'anhydride maléique (nom CTFA : PVM/MA copolymer), tels que les produits commercialisés sous les dénominations Gantrez AN-119 (masse molaire d'environ 190 000 g/mole), AN-139 (masse molaire d'environ 950 000 g/mole), AN-149 (masse molaire d'environ 1 100 000 g/mole), AN-169 (masse molaire d'environ 1 700 000 g/mole) et AN-179 (masse molaire d'environ 2 000 000 g/mole) par la société ISP ;
      (d) les copolymères de la vinylpyrrolidone avec les polyvinylalkyléthers de formule (V) suivante : 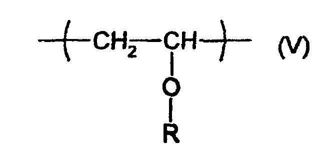 dans laquelle R est choisi parmi les groupes alkyle contenant de 1 à 7 atomes de carbone. De préférence, R est un groupe méthyle ;
      (e) les copolymères de vinylpyrrolidone et de N-vinyl lactames tel que le N-butyrolactame et le N-vinylcaprolactame ;
      (f) les copolymères de la vinylpyrrolidone avec les dérivés acryliques neutres de formule (VI) suivante : 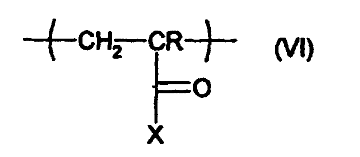
   dans laquelle R est un hydrogène ou un groupe méthyl, et X est choisi parmi les groupes oxyde d'alkyle de type OR' où R' contient de 1 à 7 atomes de carbone ; oxyde d'alkyle hydroxylé et/ou aminé de type OR₁(OH)ₙ(NR₂R₃)ₘ où n et m sont des nombres allant de 0 à 10, R₁ est un groupe alkyle contenant de 1 à 7 atomes de carbone; R₂ et R₃ sont indépendamment l'hydrogène ou un groupe alkyle tel que la somme des atomes de carbone de R₂ et R₃ aille de 1 à 7; amine primaire, secondaire ou tertiaire de type NR₂R₃ où R₂ et R₃ ont la signification indiquée ci-dessus.
D) les homopolymères et copolymères de vinylcaprolactame qui peuvent être choisis parmi :
   1) les polyvinylcaprolactames qui ont la formule (VII) suivante : 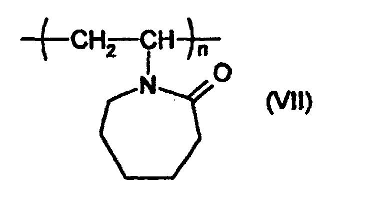
   2) les copolymères de vinylcaprolactames obtenus à partir de vinylcaprolactame et d'un ou plusieurs des monomères suivants :
      - acétate de vinyle ;
      - N-vinyl lactame tel que tel que le N-butyrolactame, le N-vinylcaprolactame et la N-vinylpyrrolidone ;
      - anhydride maléique ;
      - vinylalkyléthers de formule (V) indiquée ci-dessus;
      - dérivés acryliques neutres de formule (VI) indiquée ci-dessus.

   Comme polymères et copolymères de ce type, on peut citer par exemple le produit commercialisé sous la dénomination Luviskol Plus par la société BASF et le produit commercialisé sous la dénomination H2OLD EP-1 par la société ISP.
E) les homopolymères et copolymères de polyvinylméthyléther qui peuvent être choisis parmi :
   1) les polyvinylméthyléthers de formule (V) indiquée ci-dessus ;
   2) les copolymères obtenus à partir de vinylméthyléther et d'un ou plusieurs des monomères suivants :
      - vinylalkyléthers de formule (V) indiquée ci-dessus,
      - acétate de vinyle ;
      - N-vinyl lactame tel que le N-butyrolactame, le N-vinylcaprolactame et la N-vinylpyrrolidone ;
      - anhydride maléique ;
      - dérivés acryliques neutres de formule (VI) indiquée ci-dessus.

   Comme polymères et copolymères de ce type, on peut citer par exemple les produits commercialisés sous les dénominations Gantrez (nom CTFA : PVM/MA copolymer), et particulièrement Gantrez AN-119 (masse molaire d'environ 190 000 g/mole), AN-139 (masse molaire d'environ 950 000 g/mole), AN-149 (Masse molaire ≈ 1 100 000 g/mole), AN-169 (masse molaire d'environ 1 700 000 g/mole) et AN-179 (masse molaire d'environ 2 000 000 g/mole) par la société ISP.
F) les homopolymères et copolymères acryliques neutres, notamment ceux ayant une masse molaire allant de 10 000 g/mole à 5 000 000 g/mole. Ils peuvent être choisis parmi :
   1) les polymères acryliques hydrosolubles neutres ayant la formule (IX) suivante : 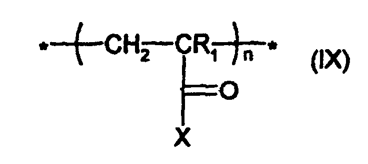 dans laquelle R₁ est un hydrogène ou un groupe méthyl, et X est choisi parmi (a) les groupes alkylaminés ou (b) les groupes oxydes d'alkyle hydroxylé et/ou aminé.
      Les polymères avec des groupes (a) alkylaminés sont des composés de formule (IX) où X = NR₂R₃ tels que le polymère acrylique correspondant soit hydrosoluble, R₂ et R₃ étant indépendamment un hydrogène ou un groupe alkyle tel que la somme des atomes de carbone de R₂ et R₃ aille de 1 à 7. Comme polymères de ce type, on peut citer notamment les polyacrylamides où R₁, R₂ et R₃ sont un hydrogène ; les polyméthylacrylamides où R₁ est un groupe méthyl et R₂ et R₃ sont un hydrogène ; les poly N-méthylacrylamides où R₁ et R₂ sont un hydrogène et R₃ est un groupe méthyl ; les poly N,N'-diméthylacrylamides où R₁ est un hydrogène et R₂ et R₃ sont un groupe méthyle ; les poly-N-éthylacrylamides où R₁ et R₂ sont un hydrogène et R₃ est un groupe éthyl ; les poly-N-isopropylacrylamides où R₁ et R₂ sont un hydrogène et R₃ est un groupe isopropyle.
      Comme polymère de ce type, on peut citer le polyacrylamide commercialisé sous la dénomination Superfloc N300 LMW par la société Cytec.
      Les polymères avec des groupes (b) oxydes d'alkyle hydroxylé et/ou aminé sont des composés de formule (IX) dans laquelle X = OR₂(OH)ₙ(NR₃R₄)ₘ où n et m sont des nombres allant de 0 à 10, R₂ est un groupe alkyle contenant de 1 à 7 atomes de carbone; R₃ et R₄ sont indépendamment l'hydrogène ou un groupe alkyle tel que la somme des atomes de carbone de R₃ et R₄ aille de 1 à 7, ces groupes étant tels que le dérivé acrylique correspondant soit hydrosoluble.
      Comme polymère de ce type, on peut citer le polyméthacrylate de glycéryle, commercialisé sous la dénomination Lubrajel CG par la société Guardian.
   2) les copolymères d'un dérivé acrylique hydrosoluble et neutre de formule (IX) tel précédemment décrit et d'un ou plusieurs monomère(s) neutre(s) suivants :
      - acétate de vinyle ;
      - N-vinyl lactame tel que le N-butyrolactame, le N-vinylcaprolactame et la N-vinylpyrrolidone ;
      - anhydride maléique ;
      - vinylalkyléthers de formule (V) indiquée ci-dessus ;
      - dérivé acrylique neutre de formule (VI) indiquée ci-dessus.
G) les alkyl-C₁-C₂-celluloses et leurs dérivés neutres, notamment ceux ayant une masse molaire allant de 10 000 g/mole à 5 000 000 g/mole. Ils peuvent être notamment choisis parmi l'hydroxyéthylcellulose comme le produit commercialisé sous les dénominations Natrosols 250 LR et 250 HHR par la société Aqualon ; l'éthylhydroxyéthylcellulose comme les produits commercialisés sous les dénominations Elfacos CD 481 et CD 411 par la société Akzo Nobel ; la méthylcellulose et les methylhydroxyalkylcelluloses comme le produit commercialisé sous la dénomination Methocel A4C par la société Dow Chemical et les produits commercialisés sous les dénominations Benecel par la société Hercules.
H) les alkyl-C₁-C₃-guar ou hydroxyalkyl-C₁-C₃-guar, notamment ceux ayant une masse molaire allant de 10 000 g/mole à 5 000 000 g/mole. On peut citer en l'hydroxypropylguar comme le produit commercialisé sous la dénomination Jaguar HP-105 par la société Rhodia.

Selon l'invention, le ou les polymères non ioniques hydrosolubles peuvent représenter une quantité de 0,01 à 20 % en poids, de préférence de 0,05 à 10 % en poids et plus particulièrement de 0,1 à 5 % en poids par rapport au poids total de la composition.

Ces nanoémulsions comprennent au moins un lipide amphiphile choisi parmi les lipides amphiphiles non ioniques, les lipides amphiphiles anioniques tels que définis ci-dessus et leurs mélanges.

Les lipides amphiphiles non ioniques de l'invention sont préférentiellement choisis parmi :
1/ les tensioactifs siliconés,
2/ les lipides amphiphiles liquides à température inférieure ou égale à 45°C, choisis parmi les esters d'au moins un polyol d'au moins un acide gras comportant au moins une chaîne alkyle en C₈-C₂₂, saturée ou non saturée, linéaire ou ramifiée, et notamment insaturée ou ramifiée, le polyol étant choisi dans le groupe formé par le polyéthylèneglycol comportant de 1 à 60 unités d'oxyde d'éthylène, le sorbitan, le glycérol pouvant comporter de 2 à 30 unités d'oxyde d'éthylène, les polyglycérols comportant de 2 à 15 unités de glycérol,
3/ les esters d'acide gras et de sucre et les éthers d'alcool gras et de sucre,
4/ les tensioactifs solides à une température inférieure ou égale à 45°C, choisis parmi les esters gras de glycérol, les esters gras de sorbitan et les esters gras de sorbitan oxyéthylénés, les éthers gras éthoxylés et les esters gras éthoxylés,
5/ Les copolymères blocs d'oxyde d'éthylène (A) et d'oxyde de propylène (B), et les mélanges de ces tensioactifs.

1/ Les tensioactifs siliconés utilisables selon l'invention sont des composés siliconés comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5,364,633 et US-A-5,411,744.
   De préférence, le tensioactif siliconé utilisé selon la présente invention est un composé de formule (X) : 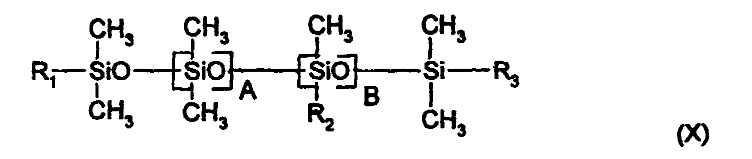 dans laquelle :
   R₁, R₂, R₃, indépendamment les uns des autres, représentent un radical alkyle en C₁-C₆ ou un radical -(CH₂)ₓ-(OCH₂CH₂)_{y}-(OCH₂CH₂CH₂)_{z}-OR₄, au moins un radical R₁, R₂ ou R₃ n'étant pas un radical alkyle ; R₄ étant un hydrogène, un radical alkyle ou un radical acyle ;
   A est un nombre entier allant de 0 à 200 ;
   B est un nombre entier allant de 0 à 50 ; à la condition que A et B ne soient pas égaux à zéro en même temps ;
   x est un nombre entier allant de 1 à 6 ;
   y est un nombre entier allant de 1 à 30 ;
   z est un nombre entier allant de 0 à 5.

   Selon un mode de réalisation préféré de l'invention, dans le composé de formule (X), le radical alkyle est un radical méthyle, x est un nombre entier allant de 2 à 6 et y est un nombre entier allant de 4 à 30.
   On peut citer, à titre d'exemple de tensioactifs siliconés de formule (X), les composés de formule (XI) : 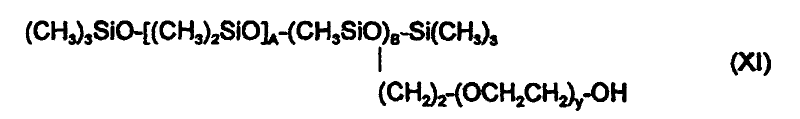 dans laquelle A est un nombre entier allant de 20 à 105, B est un nombre entier allant de 2 à 10 et y est un nombre entier allant de 10 à 20.
   On peut également citer à titre d'exemple de tensioactifs siliconés de formule (X), les composés de formule (XII)

   H-(OCH₂CH₂)_{y}-(CH₂)₃-[(CH₃)₂SiO]_{A'}-(CH₂)₃-(OCH₂CH₂)_{y}-OH (XII)

   dans laquelle A' et y sont des nombres entiers allant de 10 à 20.
   On peut utiliser notamment comme tensioactifs siliconés, ceux commercialisés par la société Dow Corning sous les dénominations DC 5329, DC 7439-146, DC 2-5695 et Q4-3667. Les composés DC 5329, DC 7439-146, DC 2-5695 sont des composés de formule (XI) où respectivement A est 22, B est 2 et y est 12 ; A est 103, B est 10 et y est 12 ; A est 27, B est 3 et y est 12.
   Le composé Q4-3667 est un composé de formule (XII) où A' est 15 et y est 13.
2/ Les lipides amphiphiles liquides à température inférieure ou égale à 45°C peuvent être notamment choisis parmi :
   - l'isostéarate de polyéthylèneglycol de poids molaire 400 (nom CTFA : PEG-8 Isostearate), vendu sous la dénomination Prisorine 3644 par la société UNICHEMA ;
   - l'isostéarate de diglycéryle, vendu par la société SOLVAY ;
   - le laurate de polyglycérol comportant 2 unités de glycérol (polyglyceryl-2 laurate), vendu sous la dénomination Diglycerin-monolaurate par la société SOLVAY ;
   - l'oléate de sorbitan, vendu sous la dénomination SPAN 80 par la société ICI ;
   - l'isostéarate de sorbitan, vendu sous la dénomination NIKKOL SI 10R par la société NIKKO ;
   - le cocoate d'α-butylglucoside ou le caprate d'α-butylglucoside commercialisés par la société ULICE.
3/ Les esters d'acide gras et de sucre, utilisables comme lipides amphiphiles non ioniques dans la nanoémulsion selon l'invention sont de préférence solides à une température inférieure ou égale à 45°C et peuvent être choisis notamment dans le groupe comprenant les esters ou les mélanges d'esters d'acide gras en C₈-C₂₂ et de sucrose, de maltose, de glucose ou de fructose, et les esters ou les mélanges d'esters d'acide gras en C₁₄-C₂₂ et de méthylglucose.
   Les acides gras en C₈-C₂₂ ou en C₁₄-C₂₂ formant le motif gras des esters utilisables dans la nanoémulsion de l'invention comportent une chaîne alkyle linéaire saturée ou non saturée, ayant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.
   On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta F50, F70, F110, F160 ayant respectivement un HLB (Hydrophilic Lipophilic Balance) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450. On peut citer aussi les monoesters de glucose ou de maltose tels que l'o-hexadécanoyle-6-D-glucoside de méthyle et l'o-hexadécanoyle-6-D-maltoside.
   Les éthers d'alcool gras et de sucre, utilisables comme lipides amphiphiles non ioniques dans la nanoémulsion selon l'invention sont solides à une température inférieure ou égale à 45°C et peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.
   Les alcools gras en C₈-C₂₂ ou en C₁₄-C₂₂ formant le motif gras des éthers utilisables dans la nanoémulsion de l'invention comportent une chaîne alkyle linéaire saturée ou non saturée, ayant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle, et leurs mélanges tels que cétéaryle.
   A titre d'exemple d'éthers d'alcool gras et de sucre, on peut citer les alkylpolyglucosides tels que le décylglucoside et le laurylglucoside commercialisés par exemple par la société Henkel sous les dénominations respectives de Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidylglucoside, par exemple sous la forme du mélange d'alcools arachidique et béhénique et d'arachidylglucoside, commercialisé sous la dénomination Montanov 202 par la société Seppic.
   On utilise plus particulièrement comme lipide amphiphile non ionique de ce type, le monostéarate de sucrose, le distéarate de sucrose, le tristéarate de sucrose et leurs mélanges, le distéarate de méthyl glucose et de polyglycérol-3 et les alkylpolyglucosides.
4/ Les esters gras de glycérol, utilisables comme lipides amphiphiles non ioniques dans la nanoémulsion selon l'invention, solides à une température inférieure ou égale à 45°C, peuvent être choisis notamment dans le groupe comprenant les esters formés d'au moins un acide comportant une chaîne alkyle linéaire saturée, ayant de 16 à 22 atomes de carbone, et de 1 à 10 motifs glycérol. On peut utiliser un ou plusieurs de ces esters gras de glycérol dans la nanoémulsion de l'invention.
   Ces esters peuvent être notamment choisis parmi les stéarates, béhénates, arachidates, palmitates et leurs mélanges. On utilise de préférence des stéarates et palmitates.
   On peut citer à titre d'exemple de tensioactif utilisable dans la nanoémulsion de l'invention, les monostéarate, distéarate, tristéarate et pentastéarate de décaglycérol (10 unités de glycérol) (noms CTFA : Polyglyceryl-10 stearate, Polyglyceryl-10 distearate, Polyglyceryl-10 tristearate, Polyglyceryl-10 pentastearate) tels que les produits vendus sous les dénominations respectives Nikkol Decaglyn 1-S, 2-S, 3-S et 5-S par la société Nikko, et le monostéarate de diglycérol (nom CTFA : Polyglyceryl-2 stearate) tel que le produit vendu par la société Nikko sous la dénomination Nikkol DGMS.
   Les esters gras de sorbitan, utilisables comme lipides amphiphiles non ioniques dans la nanoémulsion selon l'invention, solides à une température inférieure ou égale à 45°C, sont choisis notamment dans le groupe comprenant les esters d'acide gras en C₁₆-C₂₂ et de sorbitan et les esters d'acide gras en C₁₆-C₂₂ et de sorbitan oxyéthylénés. Ils sont formés d'au moins un acide gras comportant au moins une chaîne alkyle linéaire saturée, ayant respectivement de 16 à 22 atomes de carbone, et de sorbitol ou de sorbitol éthoxylé. Les esters oxyéthylénés comportent généralement de 1 à 100 unités d'oxyde d'éthylène et de préférence de 2 à 40 unités d'oxyde d'éthylène (OE).
   Ces esters peuvent être notamment choisis parmi les stéarates, béhénates, arachidates, palmitates, et leurs mélanges. On utilise de préférence des stéarates et palmitates.
   On peut citer à titre d'exemple d'ester gras de sorbitan et d'ester gras de sorbitan oxyéthyléné, utilisable dans la nanoémulsion de l'invention, le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, le tristéarate de sorbitan 20 OE (nom CTFA : Polysorbate 65) vendu par la société ICI sous la dénomination Tween 65.
   Les éthers gras éthoxylés solides à une température inférieure ou égale à 45°C, utilisables comme lipides amphiphiles non ioniques dans la nanoémulsion selon l'invention sont de préférence des éthers formés de 1 à 100 unités d'oxyde d'éthylène et d'au moins une chaîne d'alcool gras ayant de 16 à 22 atomes de carbone. La chaîne grasse des éthers peut être notamment choisie parmi les motifs béhényle, arachidyle, stéaryle, cétyle, et leurs mélanges tels que cétéaryle. A titre d'exemple d'éthers gras éthoxylés, on peut citer les éthers d'alcool béhénique comprenant 5, 10, 20 et 30 unités d'oxyde d'éthylène (noms CTFA : Beheneth-5, Beheneth-10, Beheneth-20, Beheneth-30), tels que les produits commercialisés sous les dénominations Nikkol BB5, BB10, BB20, BB30 par la société Nikko, et l'éther d'alcool stéarylique comprenant 2 unités d'oxyde d'éthylène (nom CTFA : Steareth-2), tel que le produit commercialisé sous la dénomination Brij 72 par la société ICI.
   Les esters gras éthoxylés solides à une température inférieure ou égale à 45°C, utilisables comme lipides amphiphiles non ioniques dans la nanoémulsion selon l'invention sont des esters formés de 1 à 100 unités d'oxyde d'éthylène et d'au moins une chaîne d'acide gras comportant de 16 à 22 atomes de carbone. La chaîne grasse des esters peut être notamment choisie parmi les motifs stéarate, béhénate, arachidate, palmitate, et leurs mélanges. A titre d'exemple d'esters gras éthoxylés, on peut citer l'ester d'acide stéarique comprenant 40 unités d'oxyde d'éthylène, tel que le produit commercialisé sous la dénomination Myrj 52 (nom CTFA : PEG-40 stearate) par la société ICI ainsi que l'ester d'acide béhénique comprenant 8 unités d'oxyde d'éthylène (nom CTFA : PEG-8 behenate), tel que le produit commercialisé sous la dénomination Compritol HD5 ATO par la société Gattefosse.
5/ Les copolymères blocs d'oxyde d'éthylène et d'oxyde de propylène, utilisables comme lipides amphiphiles non ioniques dans la nanoémulsion selon l'invention peuvent être choisis notamment parmi les copolymères blocs de formule (XIII) :

   HO(C₂H₄O)x(C₃H₆O)y(C₂H₄O)zH (XIII)

   dans laquelle x, y et z sont des nombres entiers tels que x+z va de 2 à 100 et y va de 14 à 60, et leurs mélanges, et plus particulièrement parmi les copolymères blocs de formule (V) ayant un HLB allant de 2 à 16.

Ces copolymères blocs peuvent être notamment choisis parmi les poloxamers et notamment parmi le Poloxamer 231 tel que le produit commercialisé par la société ICI sous la dénomination Pluronic L81 de formule (XIII) avec x=z=6, y=39 (HLB 2) ; le Poloxamer 282 tel que le produit commercialisé par la société ICI sous la dénomination Pluronic L92 de formule (XIII) avec x=z=10, y=47 (HLB 6) ; et le Poloxamer 124 tel que le produit commercialisé par la société ICI sous la dénomination Pluronic L44 de formule (XIII) avec x=z=11, y=21 (HLB 16).

Comme lipides amphiphiles non ioniques, on peut aussi citer les mélanges de tensioactifs non ioniques décrits dans le document EP-A-705593 incorporé ici pour référence.

Parmi les lipides amphiphiles non ioniques, on peut utiliser en particulier :
- l'isostéarate de PEG 400 ou PEG-8 Isostéarate (comportant 8 moles d'oxyde d'éthylène),
- l'isostéarate de diglycéryle,
- le monolaurate de polyglycérol comportant 2 unités de glycérol et les stéarates de polyglycérol comportant 10 unités de glycérol,
- l'oléate de sorbitan,
- l'isostéarate de sorbitan,
et leurs mélanges.

Les lipides amphiphiles anioniques utilisables dans les nanoémulsions de l'invention peuvent être choisis parmi :
1/ les esters mixtes d'acide gras ou d'alcool gras, d'acide carboxylique et de glycérol,
2/ les citrates d'alkyléther,
3/ les alkényl succinates choisis parmi les alkényl succinates alkoxylés, les alkényl succinates de glucose alkoxylés et les alkényl succinates de méthylglucose alkoxylés,
4/ les esters gras d'acide phosphoriques.

1/ Les esters mixtes d'acide gras ou d'alcool gras, d'acide carboxylique et de glycérol, utilisables comme lipides amphiphiles anioniques dans la nanoémulsion selon l'invention peuvent être choisis notamment dans le groupe comprenant les esters mixtes d'acide gras ou d'alcool gras ayant une chaîne alkyle comportant de 8 à 22 atomes de carbone, et d'alpha-hydroxyacide et/ou d'acide succinique, avec la glycérine. L'alpha-hydroxyacide peut être par exemple l'acide citrique, l'acide lactique, l'acide glycolique, l'acide malique et leurs mélanges.
   La chaîne alkyle des acides ou alcools gras dont dérivent les esters mixtes utilisables dans la nanoémulsion de l'invention peut être linéaire ou ramifiée, saturée ou non saturée. Il peut s'agir notamment de chaînes stéarate, isostéarate, linoléate, oléate, béhénate, arachidonate, palmitate, myristate, laurate, caprate, isostéaryle, stéaryle, linoléyle, oléyle, béhényle, myristyle, lauryle, capryle et leurs mélanges.
   On peut citer, à titre d'exemple d'esters mixtes utilisables dans la nanoémulsion de l'invention, l'ester mixte de glycérine et du mélange d'acides citrique, lactique, linoléique et oléique (nom CTFA : Glyceryl citrate/lactate/linoleate/oleate) commercialisé par la société Hüls sous la dénomination Imwitor 375 ; l'ester mixte d'acide succinique et d'alcool isostéarylique avec la glycérine (nom CTFA : Isostéaryl diglycéryl succinate) commercialisé par la société Hüls sous la dénomination Imwitor 780 K ; l'ester mixte d'acide citrique et d'acide stéarique avec la glycérine (nom CTFA : Glyceryl stéarate citrate) commercialisé par la société Hüls sous la dénomination Imwitor 370 ; l'ester mixte d'acide lactique et d'acide stéarique avec la glycérine (nom CTFA : Glyceryl stearate lactate) commercialisé par la société Danisco sous la dénomination Lactodan B30 ou Rylo LA30.
2/ Les citrates d'alkyléther utilisables comme lipides amphiphiles anioniques dans la nanoémulsion selon l'invention peuvent être choisis notamment dans le groupe comprenant les monoesters, diesters ou triesters formés par l'acide citrique et au moins un alcool gras oxyéthyléné, comportant une chaîne alkyle linéaire ou ramifiée, saturée ou non saturée, ayant de 8 à 22 atomes de carbone, et comportant de 3 à 9 groupes éthoxylés, et leurs mélanges. On peut en effet utiliser un mélange d'un ou plusieurs de ces citrates dans la nanoémulsion de l'invention.
   Ces citrates peuvent être par exemple choisis parmi les mono-, di- et tri-esters d'acide citrique et d'alcool laurique éthoxylé, comportant de 3 à 9 groupes éthoxylés, commercialisés par la société Witco sous la dénomination Witconol EC, en particulier le Witconol EC 2129 qui est majoritairement un dilaureth-9 citrate, et le Witconol EC 3129 qui est majoritairement un trilaureth-9 citrate.
   Les citrates d'alkyléther, utilisés comme lipides amphiphiles anioniques sont de préférence employés sous forme neutralisée à un pH d'environ 7, l'agent de neutralisation étant choisi parmi les bases inorganiques telles que la soude, la potasse, l'ammoniac, et les bases organiques telles que la mono-, di- et triéthanolamine, l'aminométhylpropanediol-1,3, la N-méthylglucamine, les acides aminés basiques comme l'arginine et la lysine, et leurs mélanges.
3/ Les alkényl succinates utilisables comme lipides amphiphiles anioniques dans la nanoémulsion de l'invention sont notamment des dérivés éthoxylés et/ou propoxylés et ils sont de préférence choisis parmi les composés de formules (XIV) ou (XV) :

   HOOC-(HR)C-CH₂-COO-E (XIV)

   HOOC-(HR)C- CH₂-COO-E-O-CO-CH₂-C(HR')-COOH (XV)

   dans lesquelles :
   - les radicaux R et R' sont choisis parmi les radicaux alcoylène, linéaires ou ramifiés, comportant de 6 à 22 atomes de carbone,
   - E est choisi parmi les chaînes oxyéthylénées de formule (C₂H₄O)ₙ dans laquelle n va de 2 à 100, les chaînes oxypropylénées de formule (C₃H₆O)ₙ, dans laquelle n' va de 2 à 100, les copolymères statistiques ou séquencés comprenant des chaînes oxyéthylénées de formule (C₂H₄O)ₙ et des chaînes oxypropylénées de formule (C₃H₆O)ₙ' telles que la somme de n et n' va de 2 à 100, les groupements glucoses oxyéthylénés etlou oxypropylénés comportant en moyenne de 4 à 100 motifs oxyéthylénés et/ou oxypropylénés répartis sur l'ensemble des fonctions hydroxyle, les groupements méthyl glucoses oxyéthylénés et/ou oxypropylénés comportant en moyenne de 4 à 100 motifs oxyéthylénés et/ou oxypropylénés répartis sur l'ensemble des fonctions hydroxyle.

   Dans les formules (XIV) et (XV), n et n' sont des valeurs moyennes et ne sont donc pas forcément des entiers. On choisit avantageusement pour n une valeur allant de 5 à 60 et encore plus préférentiellement de 10 à 30.
   Avantageusement, le radical R et/ou R' est choisi parmi les radicaux alcoylène linéaires comportant de 8 à 22 et de préférence de 14 à 22 atomes de carbone. Il peut s'agir par exemple du radical hexadécényl comportant 16 atomes de carbone ou du radical octadécényl comportant 18 atomes de carbone.
   Les composés de formules (XIV) et (XV) décrits ci-dessus dans lesquels E est choisi parmi les chaînes oxyéthylénées, les chaînes oxypropylénées et les copolymères comprenant des chaînes oxyéthylénées et des chaînes oxypropylénées, peuvent être préparés conformément à la description qui est donnée dans les documents WO-A-94/00508, EP-A-107199 et GB-A-2131820 incorporés ici pour référence.
   La fonction acide -COOH des lipides amphiphiles anioniques de formules (XIV) et (XV) se trouve en général dans la nanoémulsion de l'invention, sous forme neutralisée par un agent de neutralisation, l'agent de neutralisation étant choisi par exemple parmi les bases inorganiques telles que la soude, la potasse, l'ammoniac, et les bases organiques telles que la mono-, dl- et tri-éthanalamine, l'aminométhylpropanediol-1,3, la N-méthyl-glucamine, les acides aminés basiques comme l'arginine et la lysine, et leurs mélanges.
   A titre d'exemple de lipide amphiphile anionique de ce type, utilisable dans la nanoémulsion de l'invention, on peut citer l'hexadécényl succinate 18 OE (composé de formule XIV avec R=hexadécényl, E=(C₂H₄O)ₙ, n=18), l'hexadécényl succinate 45 OE (composé de formule XIV avec R=hexadécényl, E=(C₂H₄O)ₙ, n=45), le dihexadécényl succinate 18 OE (composé de formule XV avec R=R'=hexadécényl, E=(C₂H₄O)ₙ, n=18), le dihexadécényl succinate de glucose 10 OE (composé de formule XV avec R=R'=hexadécényl, E= glucose oxyéthyléné comportant 10 groupes oxyéthylénés), le dihexadécényl succinate de glucose 20 OE (composé de formule XV avec R=R'=hexadécényl, E=glucose oxyéthyléné comportant 20 groupes oxyéthylénés), le dioctadécényl-succinate de méthyl glucose 20 OE (composé de formule XV avec R=R'=octadécényl, E= méthyl glucose oxyéthyléné comportant 20 groupes oxyéthylénés), et leurs mélanges.
4/ Les esters gras d'acide phosphorique, et leurs dérivés oxyéthylénés, utilisables comme lipides amphiphiles anioniques dans la nanoémulsion selon l'invention peuvent être choisis notamment dans le groupe comprenant les esters formés d'acide phosphorique et d'au moins un alcool comportant une chaîne alkyle linéaire ou ramifiée, saturée ou non saturée, ayant de 8 à 22 atomes de carbone et les esters formés d'acide phosphorique et d'au moins un alcool éthoxylé, comportant une chaîne alkyle linéaire ou ramifiée, saturée ou non saturée, ayant de 8 à 22 atomes de carbone et comportant de 2 à 40 groupes oxyéthylénés, leurs sels et leurs mélanges. On peut en effet utiliser un mélange d'un ou plusieurs de ces esters d'acide phosphorique dans la nanoémulsion de l'invention.

Ces esters peuvent être notamment choisis parmi les esters d'acide phosphorique et d'alcools en C9-C15 ou leurs sels, tels que le sel de potassium d'alkyl-C9-15-phosphate commercialisé sous la dénomination Arlatone MAP par la société ICI ; les esters d'acide phosphorique et d'alcools stéarylique et/ou isostéarylique, tels que le phosphate d'alcools stéarylique/isostéarylique (nom CTFA : Octyldecyl phosphate), commercialisé sous la dénomination Hostaphat CG120 par la société Hoechst Celanese ; les esters d'acide phosphorique et d'alcool cétylique, et leurs dérivés oxyéthylénés, tels que le produit commercialisé sous la dénomination Crodafos CES (mélange d'alcool cétéarylique, de dicétyl phosphate et de ceteth-10 phosphate) par la société Croda ; les esters d'acide phosphorique et d'alcool tridécylique, et leurs dérivés oxyéthylénés, tels que le produit commercialisé sous la dénomination Crodafos T10 (nom CTFA : Trideceth-10 Phosphate) par la société Croda. Les dérivés oxyéthylénés d'acide phosphorique et d'alcool gras peuvent être préparés conformément à la description donnée dans la demande de brevet WO-A-96/14145 dont le contenu est Incorporé à la présente demande par référence.

Ces esters gras d'acide phosphorique sont de préférence employés sous forme neutralisée à un pH d'environ 7, l'agent de neutralisation étant choisi parmi les bases inorganiques telles que la soude, la potasse, l'ammoniac, et les bases organiques telles que la mono-, di- et tri-éthanolamine, l'aminométhylpropanediol-1,3, la N-méthylglucamine, les acides aminés basiques comme l'arginine et la lysine, et leurs mélanges.

Selon son caractère plus hydrophile ou plus lipophile, le lipide amphiphile non ionique ou anionique peut être introduit dans la phase aqueuse ou dans la phase huileuse de la nanoémulsion. La quantité de lipide amphiphile va de 0,2 à 15 % en poids et de préférence de 1 à 10 % en poids par rapport au poids total de la nanoémulsion.

Le rapport en poids de la quantité de la phase huileuse sur la quantité de lipide amphiphile va de 1,2 à 10 et, de préférence, de 1,2 à 6. On entend ici par "quantité de phase huileuse" la quantité totale des constituants de cette phase sans inclure la quantité de lipide amphiphile.

Selon une forme particulière de réalisation de l'invention, la nanoémulsion de l'invention peut contenir en outre un ou plusieurs lipides amphiphiles ioniques (anioniques ou cationiques) additionnels. Leur ajout, comme additif, peut améliorer encore la stabilité de la dispersion. De préférence, les lipides anioniques additionnels peuvent être présents dans les nanoémulsions à base de lipides neutres ou anioniques, et les lipides additionnels cationiques dans les nanoémulsions à base de lipides neutres.

Ainsi, les lipides amphiphiles anioniques additionnels pouvant être utilisés dans les nanoémulsions de l'invention sont choisis de préférence parmi :
- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et disodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides ;
- les dérivés alkylsulfoniques notamment de formule(XVI) : 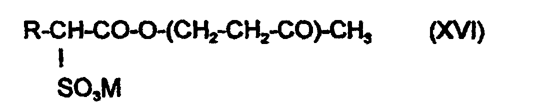
dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium ;
et leurs mélanges.

Les lipides amphiphiles cationiques pouvant être utilisés dans les nanoémulsions de l'invention sont choisis, de préférence, dans le groupe formé par les sels d'ammonium quaternaire, les amines grasses et leurs sels.

Les sels d'ammonium quaternaires sont par exemple :
- ceux qui présentent la formule générale (XVII) suivante : 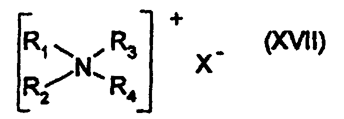 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium, comme par exemple celui de formule (XVIII) suivante: 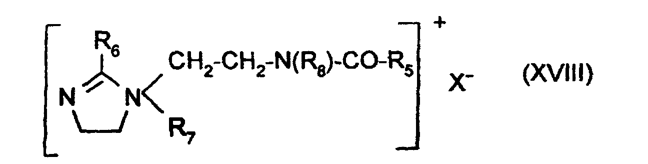 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,

Parmi les sels d'ammonium quaternaire de formule (XVII), on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK. Le chlorure de béhényltriméthylammonium est le sel d'ammonium quaternaire le plus particulièrement préféré.
- les sels de diammonium quaternaire de formule (XIX) : 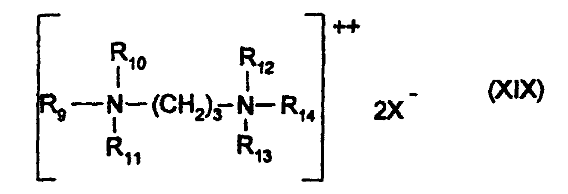 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester

Les sels d'ammonium quaternaire contenant au moins une fonction ester utilisables selon l'invention sont par exemple ceux de formule (XX) suivante : 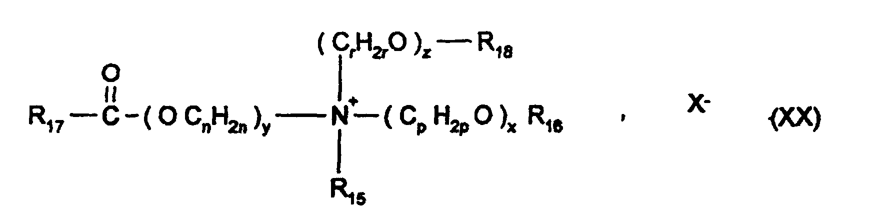 dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
   - le radical 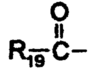
   - les radicaux R₂₀ hydrocarbonés en C₁-C₂₂ linéaires ou ramifiés, saturés ou insaturés,
   - l'atome d'hydrogène,
- R₁₈ est choisi parmi :
   - le radical 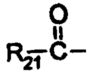
   - les radicaux R₂₂ hydrocarbonés en C₁-C₆ linéaires ou ramifiés, saturés ou insaturés,
   - l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 10 ;
- x et z, identiques ou différents, sont des entiers valant de 0 à 10 ;
- X⁻ est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.
De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

Dans la formule (XX), l'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester. L'anion X⁻ est encore plus particulièrement le chlorure ou le méthylsulfate.

On utilise plus particulièrement les sels d'ammonium de formule (XX) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 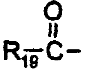
   - les radicaux méthyle, éthyle ou hydrocarbonés en C₁₄-C₂₂
   - l'atome d'hydrogène ;
- R₁₈ est choisi parmi :
   - le radical 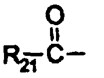
   - l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.
Avantageusement, les radicaux hydrocarbonés sont linéaires.

On peut citer par exemple comme composés de formule (XX) les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents. Ces produits sont obtenus par exemple par estérification directe de la triéthanolamine, de la triisopropanolamine, d'alkyldiéthanolamine ou d'alkyldiiso-propanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Quand elle contient des sels d'ammonium, la composition selon l'invention contient de préférence un mélange de sels de mono, di et triester d'ammonium quatemaire avec une majorité en poids de sels de diester.

Comme mélange de sels d'ammonium, on peut utiliser par exemple le mélange contenant 15 à 30 % en poids de méthylsulfate d'acyloxyéthyl dihydroxyéthyl méthyl ammonium, 45 à 60% de méthylsulfate de diacyloxyéthyl hydroxyéthyl méthyl ammonium et 15 à 30% de méthylsulfate de triacyloxyéthyl méthyl ammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4 137 180.

Lorsque la nanoémulsion contient un ou plusieurs lipides amphiphiles additionnels ioniques, ils sont présents dans les nanoémulsions de l'invention, de préférence, dans des concentrations allant de 0,01 à 10 % en poids par rapport au poids total de la nanoémulsion et plus particulièrement de 0,2 à 1 % en poids.

La phase huileuse de la nanoémulsion selon l'invention comprend au moins une huile. Les huiles pouvant être utilisées dans les nanoémulsions de l'invention sont choisies préférentiellement dans le groupe formé par :
- les huiles d'origine animale ou végétale, formées par des esters d'acides gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone, en particulier alkyle ou alkényle, par exemple, l'huile de Purcellin ou la cire liquide de jojoba ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- les huiles de synthèse telles que l'huile de parléam, les polyoléfines et les esters d'acides carboxyliques liquides ;
- les huiles minérales telles que l'hexadécane, l'isohexadécane et l'huile de paraffine ;
- les huiles halogénés, notamment des fluorocarbures tels que des fluoramines par exemple la perfluorotributylamine, des hydrocarbures fluorés, par exemple le perfluorodécahydronaphtalène, des fluoroesters et des fluoroéthers ;
- les huiles de silicone volatiles ou non volatiles.

Les polyoléfines utilisables comme huiles de synthèse sont en particulier les poly-α-oléfines et plus particulièrement celles de type polybutène, hydrogéné ou non, et de préférence polyisobutène, hydrogéné ou non.

Les esters d'acides carboxyliques liquides utilisables comme huiles de synthèse, peuvent être des esters d'acides mono-, di-, tri- ou tétra-carboxyliques. Le nombre total de carbone des esters est généralement supérieur ou égal à 10 et de préférence inférieur à 100 et plus particulièrement inférieur à 80. Ce sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant généralement supérieur ou égal à 10. On peut également utiliser les esters d'acides di- ou tri- carboxyliques en C4-C22 et d'alcools en C1-C22 et les esters d'acides mono-, di-ou tri-carboxyliques et d'alcools di-, tri-, tétra- ou penta-hydroxylés en C2-C26.

Parmi les esters cités ci-dessus, on préfère utiliser les palmitates d'alkyle tels que le palmitate d'éthyle, le palmitate d'isopropyle, le palmitate d'éthyl-2-héxyle, le palmitate de 2-octyldécyle ; les myristates d'alkyle tels que le myristate d'isopropyle, le myristate de butyle, le myristate de cétyle, le myristate de 2-octyldodécyle ; les stéarates d'alkyle tel que le stéarate d'hexyle, le stéarate de butyle, le stéarate d'isobutyle ; les malates d'alkyle tels que le malate de dioctyle ; les laurates d'alkyle tels que le laurate d'hexyle et le laurate de 2-hexyldécyle ; l'isononanate d'isononyle ; l'octanoate de cétyle.

Les nanoémulsions conformes à l'invention comportent une quantité de phase huileuse (huile et autres corps gras hormis le lipide amphiphile) allant de préférence, de 2 à 40 % en poids par rapport au poids total de la nanoémulsion et plus particulièrement de 4 à 30 % en poids et préférentiellement de 4 à 20 % en poids.

Les nanoémulsions conformes à la présente invention peuvent contenir des solvants, notamment pour améliorer, si nécessaire, la transparence de la composition.

Ces solvants sont choisis de préférence dans le groupe formé par :
- les alcools inférieurs en C₁-C₈ tels que l'éthanol ;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.
- les sucres tels que le glucose, le fructose, le maltose, le lactose, le sucrose.

Ces additifs peuvent être utilisés en mélange. Lorsqu'ils sont présents dans la nanoémulsion de l'invention, ils peuvent être utilisés à des concentrations allant de préférence de 0,01 à 30 % en poids par rapport au poids total de la nanoémulsion, et mieux de 5 à 20 % en poids par rapport au poids total de la nanoémulsion. La quantité d'alcool(s) et/ou de sucre(s) va de préférence de 5 à 20 % en poids par rapport au poids total de la nanoémulsion et la quantité de glycol(s) va de préférence de 5 à 15 % en poids par rapport au poids total de la nanoémulsion.

Le procédé de préparation d'une nanoémulsion telle que définie ci-dessus consiste à mélanger la phase aqueuse et la phase huileuse, sous agitation vive, à une température ambiante inférieure à 45°C, à effectuer une étape d'homogénéisation haute pression à une pression supérieure à 5.10⁷ Pa et à ajouter le polymère utilisé selon l'invention. Selon un mode préféré de réalisation de l'invention, on effectue ensuite encore une étape d'homogénéisation haute pression à une pression supérieure à 5.10⁷ Pa. L'homogénéisation haute pression est réalisée de préférence à une pression allant de 6.10⁷ à 18.10⁷ Pa. Le cisaillement va de préférence de 2.10⁶ s⁻¹ à 5.10⁸ s⁻¹ et mieux de 1.10⁸ s⁻¹ à 3.10⁸ s⁻¹ (s⁻¹ signifie seconde⁻¹). Un tel procédé permet de réaliser, à température ambiante, des nanoémulsions compatibles avec des composés actifs thermosensibles, et pouvant contenir des huiles et notamment des parfums qui renferment des corps gras, sans les dénaturer.

Les nanoémulsions définies ci-dessus peuvent être utilisées dans tout domaine où ce type de composition est utile. Elles peuvent constituer notamment des compositions à usage topique et en particulier des compositions cosmétiques ou dermatologiques selon le type d'actifs et la quantité de ces actifs qu'elles comportent Elles peuvent aussi être utilisées comme supports ophtalmiques. Elles peuvent en outre constituer dans le domaine pharmaceutique le support d'une composition pharmaceutique qui peut être administrée par voie orale, parentérale ou transcutanée.

Une telle composition à usage topique, pharmaceutique ou ophtalmique contient un milieu physiologiquement acceptable, c'est-à-dire compatible avec la peau, les muqueuses, le cuir chevelu, les yeux et/ou les cheveux.

L'invention a aussi pour objet un support ophtalmique, caractérisé en ce qu'il contient une nanoémulsion telle que définie précédemment.

L'invention a aussi pour objet une composition pharmaceutique, caractérisée en ce qu'elle contient une nanoémulsion telle que définie précédemment.

Un autre objet de l'invention consiste en une composition cosmétique ou dermatologique, caractérisée en ce que qu'elle est constituée d'une nanoémulsion ou comprend une nanoémulsion telle que définie précédemment.

Les compositions de l'invention peuvent contenir des adjuvants et notamment des actifs hydrosolubles ou liposolubles, ayant une activité cosmétique ou dermatologique. Les actifs liposolubles sont dans les globules huileux de l'émulsion, tandis que les actifs hydrosolubles sont dans la phase aqueuse de l'émulsion. On peut citer, à titre d'exemples d'actif, les vitamines et leurs dérivés telles que la vitamine E et ses esters tels que l'acétate de vitamine E, la vitamine C et ses esters, les vitamines B, la vitamine A alcool ou rétinol et ses esters tels que le palmitate de vitamine A, la vitamine A acide ou acide rétinoïque et ses dérivés, les provitamines telles que le panthénol, la niacinamide, l'ergocalciférol, les anti-oxydants, les huiles essentielles, les humectants, les filtres solaires, les agents hydratants, les protéines, les céramides et les pseudocéramides, la DHEA et ses dérivés et précurseurs biologiques. Comme adjuvants, on peut citer aussi les séquestrants, les adoucissants, les matières colorantes (pigments ou colorants) et les parfums.

Comme actifs ophtalmiques, on peut citer par exemple les agents anti-glaucome, tels que le betaxolol ; les antibiotiques tels que l'acyclovir ; les antiallergiques ; les agents anti-inflammatoires tels que l'ibuprofène et ses sels, le diclofénac et ses sels, l'indométhacine ; les agents antiviraux.

Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

La nanoémulsion de l'invention peut être par exemple utilisée pour le soin, le traitement, le maquillage de la peau, du visage et/ou du cuir chevelu.

L'invention a donc aussi pour objet l'utilisation cosmétique de la nanoémulsion telle que définie ci-dessus pour le soin, le traitement et/ou le maquillage de la peau, du visage et/ou du cuir chevelu.

En outre, la nanoémulsion de l'invention peut aussi être utilisée pour le soin et/ou le traitement des cheveux. Elle permet d'obtenir un dépôt d'huile sur les cheveux, ce qui rend ceux-ci plus brillants, plus résistants au coiffage, sans toutefois les alourdir. Elle permet aussi, en prétraitement d'améliorer les effets de la coloration ou de la permanente.

L'invention a donc aussi pour objet l'utilisation cosmétique de la nanoémulsion telle que définie ci-dessus pour le soin et/ou le traitement des cheveux.

La nanoémulsion selon l'invention permet notamment une bonne hydratation de la peau, des muqueuses et/ou du cuir chevelu, et est particulièrement adaptée au traitement de la peau sèche.

Un autre objet de l'invention est donc un procédé cosmétique de soin et/ou d'hydratation de la peau, des muqueuses et/ou du cuir chevelu, caractérisé en ce qu'on applique sur la peau, les muqueuses et/ou le cuir chevelu une nanoémulsion telle que définie ci-dessus.

L'invention porte également sur l'utilisation de la nanoémulsion selon l'invention pour la fabrication d'une composition destinée au traitement de la peau sèche.

Enfin, l'invention porte aussi sur l'utilisation de la nanoémulsion selon l'invention pour la fabrication d'une composition ophtalmologique.

Les exemples qui suivent, permettront de mieux comprendre l'invention, sans toutefois présenter un caractère limitatif. Les quantités indiquées sont en % en poids sauf mention contraire.

### Exemple 1 : Sérum transparent gélifié

| | | |
|---|---|---|
| A. | Isostéarate de PEG 400 | 4,5 % |
| | Acylglutamate disodique | 0,5 % |
| | Myristate d'isopropyle | 5 % |
| | Stéarate d'isocétyle | 10 % |
| | | |
| B. | Dipropylène glycol | 10 % |
| | Glycérine | 5 % |
| | Eau distillée | 32,5 % |
| | | |
| C. | Poly-oxyde d'éthylène de masse molaire 300 000 g/mole | 3 % |
| | Eau distillée | 29,5 % |

Mode opératoire : La nanoémulsion est préparée à l'homogénéiseur haute-pression à partir des phases A et B. La phase C est préparée par agitation du polymère dans l'eau à 80°C pendant 4 heures. Après refroidissement à la température ambiante, la phase C est introduite sous agitation à la défloculeuse dans la nanoémulsion.

On obtient une crème ayant une turbidité de 288 NTU, une viscosité de 1,1 Pa.s (à 200s⁻¹). Cette crème est stable et s'étale bien sur la peau.

### Exemple 2 : Sérum transparent gélifié

| | | |
|---|---|---|
| A. | Isostéarate de PEG 400 | 4,5 % |
| | Acylglutamate disodique | 0,5 % |
| | Myristate d'isopropyle | 5 % |
| | Stéarate d'isocétyle | 10 % |
| | | |
| B. | Dipropylène glycol | 10 % |
| | Glycérine | 5 % |
| | Eau distillée | 32,5 % |
| | | |
| C. | Hydroxypropylguar (Jaguar HP-105) | 0,8 % |
| | Eau distillée | 31,7 % |

Mode opératoire : La nanoémulsion est préparée à l'homogénéiseur haute-pression à partir des phases A et B. La phase C est préparée par agitation du polymère dans l'eau à 25°C pendant 4 heures puis est introduite sous agitation à la défloculeuse dans la nanoémulsion. L'ensemble est passée à l'homogénéiseur haute-pression dans les mêmes conditions.

On obtient une composition transparente ayant une turbidité de 250 NTU, une viscosité de 0,9 Pa.s (mobile 3, à 200s⁻¹) et un pH d'environ égal à 7. Cette composition s'étale bien sur la peau et est agréable à utiliser.

### Exemple 3 :

| | | |
|---|---|---|
| A. | Isostéarate de PEG 400 | 4,5 % |
| | Acylglutamate disodique | 0,5 % |
| | Myristate d'isopropyle | 5 % |
| | Stéarate d'isocétyle | 10 % |
| | | |
| B. | Dipropylène glycol | 10 % |
| | Glycérine | 5 % |
| | Eau distillée | 44,9 % |
| | Conservateur | 0,1 % |
| | | |
| C. | Natrosol 250HHR | 0,5 % |
| | Eau distillée | 19,5 % |

La nanoémulsion est préparée à l'homogénéiseur haute-pression à partir des phases A et B. La phase C est préparée par agitation du polymère dans l'eau à 25°C pendant 4 heures, et elle est ensuite introduite sous agitation à la défloculeuse dans la nanoémulsion. La turbidité de la composition transparente obtenue est de 205 NTU et sa viscosité de 0,47 Pa.s (mobile 2, Vitesse de cisaillement = 200 s⁻¹).

### Exemple comparatif :

| | | |
|---|---|---|
| A. | Isostéarate de PEG 400 | 4,5 % |
| | Acylglutamate disodique | 0,5 % |
| | Myristate d'isopropyle | 5 % |
| | Stéarate d'isocétyle | 10 % |
| | | |
| B. | Dipropylène glycol | 10 % |
| | Glycérine | 5 % |
| | Eau distillée | 45 % |
| | | |
| C. | Carbopol 980 | 0,26 % |
| | Triéthanolamine | 0,39 % |
| | Eau distillée | 19,35 % |

La nanoémulsion est préparée à partir des phases A et B à l'aide d'un homogénéisateur haute pression. La phase C est introduite sous agitation à la défloculeuse dans la nanoémulsion. La formule obtenue est blanche.

## Revendications

1. Nanoémulsion huile-dans-eau comportant une phase huileuse dispersée dans une phase aqueuse, dont les globules d'huile ont une taille moyenne en nombre inférieure à 100 nm, **caractérisée en ce qu'**elle comprend (i) au moins un lipide amphiphile choisi parmi les lipides amphiphiles non ioniques et les lipides amphiphiles anioniques, (ii) au moins un polymère non ionique hydrosoluble, totalement exempt de chaîne hydrophobe, choisi parmi les homopolymères et copolymères d'oxyde d'éthylène ayant une masse molaire égale ou supérieure à 10 000 g/mole ; les alcools polyvinyliques ; les homopolymères et copolymères de vinylpyrrolidone ; les homopolymères et copolymères de vinylcaprolactame ; les homopolymères et copolymères de polyvinylméthyléther ; les homopolymères et copolymères acryliques neutres ; les alkyl-C₁-C₂-celluloses et leurs dérivés ; les alkyl-C₁-C₃-guar ou hydroxyalkyl-C₁-C₃-guar, et (iii) au moins un lipide amphiphile ionique additionnel choisi parmi :
- les sels alcalins du dicétyl- et du dimyristylphosphate ;
- les sels alcalins du cholestérol sulfate ;
- les sels alcalins du cholestérol phosphate ;
- les lipoaminoacides et leurs sels ;
- les sels de sodium de l'acide phosphatidique ;
- les phospholipides ;
- les dérivés alkylsulfoniques ;
- les sels d'ammonium quaternaire,
- les amines grasses ;
- et leurs mélanges ;
et **en ce que** le rapport pondéral de la quantité de phase huileuse sur la quantité de lipide amphiphile va de 2 à 10.

2. Nanoémulsion selon la revendication 1, **caractérisée en ce que** le rapport pondéral de la quantité de phase huileuse sur la quantité de lipide amphiphile va de 2 à 6.

3. Nanoémulsion selon l'une quelconque des revendications précédentes, **caractérisée en ce que** les globules d'huile ont une taille moyenne en nombre allant de 20 à 80 nm.

4. Nanoémulsion selon l'une quelconque des revendications précédentes, **caractérisée en ce que** la présence de polymère augmente la viscosité de la nanoémulsion d'au moins un facteur 5.

5. Nanoémulsion selon l'une quelconque des revendications précédentes, **caractérisée en ce qu'**elle a une turbidité allant de 60 à 400 NTU.

6. Nanoémulsion selon l'une quelconque des revendications précédentes, **caractérisée en ce que** les homopolymères et copolymères d'oxyde d'éthylène sont choisis parmi :
(1) les poly-oxydes d'éthylène ayant la formule (I) suivante :
R-(CH₂-CH₂-O)ₙ-R' (I)
dans laquelle R est choisi parmi les groupes hydroxyle, méthoxy et amine, R' est un groupe méthyl ou un hydrogène, et n est un nombre allant de 220 à 230 000 ;
(2) les copolymères d'oxyde d'éthylène et d'un ou plusieurs monomères oxyalkylénés ayant la formule (II) suivante :
-(CHR-CHR'-O)- (II)
dans laquelle R et R' indépendamment l'un de l'autre sont un hydrogène ou un groupe alkyle comportant de 1 à 7 atomes de carbone, l'un au moins de R ou R' étant un groupe alkyle.

7. Nanoémulsion selon l'une quelconque des revendications précédentes, **caractérisée en ce que** les alcools polyvinyliques sont des composés de formule (III) suivante : 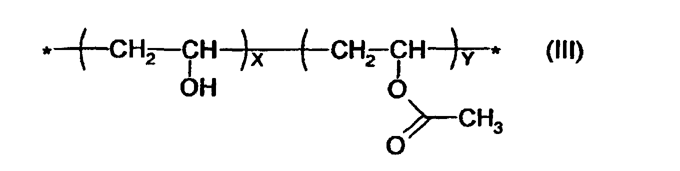 dans laquelle x est un nombre moyen exprimé en pourcentage allant de 70 à 100 ; y est un nombre moyen égal à 100 - x.

8. Nanoémulsion selon l'une quelconque des revendications précédentes, **caractérisée en ce que** les homopolymères et copolymères de vinylpyrrolidone sont choisis parmi :
- les polyvinylpyrrolidones ayant la formule (IV) suivante : 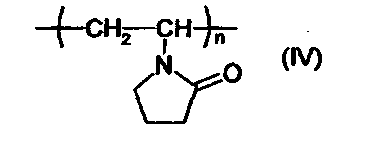
- les copolymères de vinylpyrrolidone et d'acétate de vinyle ;
- les copolymères de vinylpyrrolidone et de dérivés de vinylpyrrolidone à greffons butène ;
- les copolymères de vinylpyrrolidone et d'anhydride maléique ;
- les copolymères de la vinylpyrrolidone avec les polyvinytalkyléthers de formule (V) suivante : 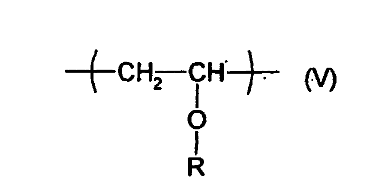
dans laquelle R est choisi parmi les groupes alkyles contenant de 1 à 7 atomes de carbone ;
- les copolymères de vinylpyrrolidone et de N-vinyl lactames ;
- les copolymères de la vinylpyrrolidone avec les dérivés acryliques neutres de formule (VI) suivante : 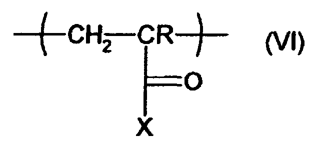
dans laquelle R est un hydrogène ou un groupe méthyl, et X est choisi parmi les groupes oxyde d'alkyle de type OR' où R' contient de 1 à 7 atomes de carbone ; oxyde d'alkyle hydroxylé et/ou aminé de type OR₁(OH)ₙ(NR₂R₃)ₘ où n et m sont des nombres allant de 0 à 10, R₁ est un groupe alkyle contenant de 1 à 7 atomes de carbone; R₂ et R₃ sont indépendamment l'hydrogène ou un groupe alkyle tel que la somme des atomes de carbone de R₂ et R₃ aille de 1 à 7 ; amine primaire, secondaire ou tertiaire de type NR₂R₃ où R₂ et R₃ ont la signification indiquée ci-dessus.

9. Nanoémulsion selon l'une quelconque des revendications précédentes, **caractérisée en ce que** les homopolymères et copolymères de vinylcaprolactame sont choisis parmi :
- les polyvinylcaprolactames qui ont la formule (VII) suivante : 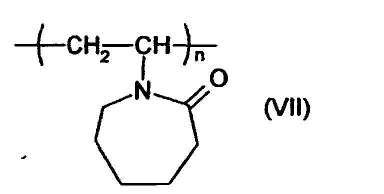
- les copolymères de vinylcaprolactames obtenus à partir de vinylcaprolactame et d'un ou plusieurs des monomères suivants :
- acétate de vinyle ;
- N-vinyl lactame ;
- anhydride maléique ;
- vinylalkyléthers de formule (V) ;
- dérivés acryliques neutres de formule (VI).

10. Nanoémulsion selon l'une quelconque des revendications précédentes, **caractérisée en ce que** les copolymères de polyvinylméthyléther sont obtenus à partir de vinylméthyléther et d'un ou plusieurs des monomères suivants :
- les vinylalkyléthers de formule (V) ;
- acétate de vinyle ;
- N-vinyl lactame ;
- anhydride maléique ;
- dérivés acryliques neutres de formule (VI).

11. Nanoémulsion selon l'une quelconque des revendications précédentes, **caractérisée en ce que** les homopolymères et copolymères acryliques neutres, sont choisis parmi :
- les polymères acryliques hydrosolubles neutres ayant la formule (IX) suivante : 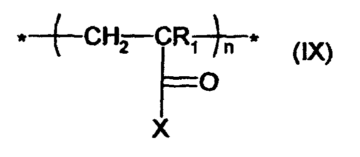
dans laquelle R₁ est un hydrogène ou un groupe méthyl, et X est choisi parmi
(a) parmi les groupes alkylaminés de type NR₂R₃, tels que le polymère acrylique correspondant soit hydrosoluble, où R₂ et R₃ sont indépendamment un hydrogène ou un groupe alkyle tel que la somme des atomes de carbone de R₂ et R₃ aille de 1 à 7 ;
(b) parmi les groupes oxydes d'alkyle hydroxylé et/ou aminé de type OR₂(OH)ₙ(NR₃R₄)ₘ où n et m sont des nombres allant de 0 à 10, R₂ est un groupe alkyle contenant de 1 à 7 atomes de carbone; R₃ et R₄ sont indépendamment l'hydrogène ou un groupe alkyle tel que la somme des atomes de carbone de R₃ et R₄ aille de 1 à 7, ces groupes étant tels que le dérivé acrylique correspondant soit hydrosoluble.
- les copolymères d'un dérivé acrylique de formule (IX) et d'un ou plusieurs monomère(s) neutre(s) suivants :
- acétate de vinyle ;
- N-vinyl lactame ;
- anhydride maléique ;
- vinylalkyléthers de formule (V) ;
- dérivé acrylique neutre de formule (VI).

12. Nanoémulsion selon l'une quelconque des revendications précédentes, **caractérisée en ce que** les alkyl-C₁-C₂-celluloses et leurs dérivés neutres sont choisis parmi l'hydroxyéthylcellulose ; l'éthylhydroxyéthylcellulose ; la méthylcellulose et les methylhydroxyalkylcelluloses.

13. Nanoémulsion selon l'une quelconque des revendications précédentes, **caractérisée en ce que** le polymère est l'hydroxypropylguar.

14. Nanoémulsion selon l'une quelconque des revendications précédentes, **caractérisée en ce que** la quantité de polymère(s) non ionique(s) hydrosoluble(s) va de 0,01 à 20 % en poids par rapport au poids total de la composition.

15. Nanoémulsion selon l'une quelconque des revendications précédentes, **caractérisée en ce que** le lipide amphiphile non ionique est choisi parmi :
1/ les tensioactifs siliconés,
2/ les lipides amphiphiles liquides à température inférieure ou égale à 45°C choisis parmi les esters d'au moins un polyol et d'au moins un acide gras comportant au moins une chaîne alkyle en C₈-C₂₂, saturée ou non saturée, linéaire ou ramifiée,
3/ les esters d'acide gras et de sucre et les éthers d'alcool gras et de sucre,
4/ les tensioactifs solides à une température inférieure ou égale à 45°C, choisis parmi les esters gras de glycérol, les esters gras de sorbitan et les esters gras de sorbitan oxyéthylénés, les éthers gras éthoxylés et les esters gras éthoxylés,
5/ les copolymères blocs d'oxyde d'éthylène et d'oxyde de propylène, et leurs mélanges.

16. Nanoémulsion selon l'une quelconque des revendications précédentes, **caractérisée en ce que** la quantité de lipide amphiphile va de 0,2 à 15 % en poids par rapport au poids total de la composition.

17. Nanoémulsion selon l'une quelconque des revendications précédentes, **caractérisée en ce que** le lipide amphiphile ionique additionnel est présent en une quantité allant de 0,01 à 10 % en poids par rapport au poids total de la composition.

18. Nanoémulsion selon l'une quelconque des revendications précédentes, **caractérisée en ce que** la quantité de phase huileuse va de 2 à 40 % en poids par rapport au poids total de la composition.

19. Nanoémulsion selon l'une quelconque des revendications précédentes, **caractérisée en ce qu'**elle constitue une composition cosmétique ou dermatologique.

20. Nanoémulsion selon l'une quelconque des revendications 1 à 18, **caractérisée en ce qu'**elle constitue un support ophtalmique.

21. Nanoémulsion selon l'une quelconque des revendications 1 à 18, **caractérisée en ce qu'**elle constitue une composition pharmaceutique.

22. Utilisation cosmétique de la nanoémulsion selon l'une quelconque des revendications 1 à 18 ou de la composition selon la revendication 19, pour le soin, le traitement et/ou le maquillage de la peau, du visage et/ou du cuir chevelu.

23. Utilisation cosmétique de la nanoémulsion selon l'une quelconque des revendications 1 à 18 ou de la composition selon la revendication 19, pour le soin et/ou le traitement des cheveux.

24. Procédé cosmétique de soin et/ou d'hydratation de la peau, des muqueuses et/ou du cuir chevelu, **caractérisé en ce qu'**on applique sur la peau, les muqueuses et/ou le cuir chevelu, une nanoémulsion selon l'une quelconque des revendications 1 à 18 ou une composition selon la revendication 19.

25. Utilisation de la nanoémulsion selon l'une quelconque des revendications 1 à 18, pour la fabrication d'une composition destinée au traitement de la peau sèche.

26. Utilisation de la nanoémulsion selon l'une quelconque des revendications 1 à 18, pour la fabrication d'une composition ophtalmologique.

27. Procédé pour épaissir une nanoémulsion huile-dans-eau ayant des globules d'huile dont la taille moyenne en nombre est inférieure à 100 nm, consistant à ajouter à ladite nanoémulsion au moins un polymère non ionique hydrosoluble, totalement exempt de chaîne hydrophobe, choisi parmi les homopolymères et copolymères d'oxyde d'éthylène ayant une masse molaire égale ou supérieure à 10 000 g/mole ; les alcools polyvinyliques ; les homopolymères et copolymères de vinylpyrrolidone ; les homopotymères et copolymères de vinylcaprolactame ; les homopolymères et copolymères de polyvinylméthyléther ; les homopolymères et copolymères acryliques neutres ; les alkyl-C₁-C₂-celluloses et leurs dérivés ; les alkyl-C₁-C₃-guar ou hydroxyalkyl-C₁-C₃-guar, la présence du polymère augmentant la viscosité de la nanoémulsion d'au moins un facteur 5 pour une concentration en polymère égale à 1 % en poids.

## Claims

1. Oil-in-water nanoemulsion comprising an oily phase dispersed in an aqueous phase, the oil globules of which have a number-average size of less than 100 nm, **characterized in that** it comprises (i) at least one amphiphilic lipid chosen from nonionic amphiphilic lipids and anionic amphiphilic lipids, (ii) at least one water-soluble nonionic polymer totally free of hydrophobic chain and chosen from homopolymers and copolymers of ethylene oxide having a molar mass equal to or greater than 10,000 g/mol; polyvinyl alcohols; homopolymers and copolymers of vinylpyrrolidone; homopolymers and copolymers of vinylcaprolactam; homopolymers and copolymers of polyvinyl methyl ether; neutral acrylic homopolymers and copolymers; C₁-C₂ alkyl celluloses and their derivatives; C₁-C₃ alkyl guar or C₁-C₃ hydroxyalkyl guar and (iii) at least one additional ionic amphiphilic lipid chosen from:
- the alkali metal salts of dicetyl and dimyristyl phosphate;
- the alkali metal salts of cholesterol sulphate;
- the alkali metal salts of cholesterol phosphate;
- lipoamino acids and their salts;
- the sodium salts of phosphatidic acid;
- phospholipids;
- alkylsulphonic derivatives;
- quaternary ammonium salts;
- fatty amines;
- and mixtures thereof;
and **in that** the ratio by weight of the amount of oily phase to the amount of amphiphilic lipid ranges from 2 to 10.

2. Nanoemulsion according to Claim 1, **characterized in that** the ratio by weight of the amount of oily phase to the amount of amphiphilic lipid ranges from 2 to 6.

3. Nanoemulsion according to any one of the preceding claims, **characterized in that** the oil globules have a number-average size ranging from 20 to 80 nm.

4. Nanoemulsion according to any one of the preceding claims, **characterized in that** the presence of polymer increases the viscosity of the nanoemulsion by at least a factor of 5.

5. Nanoemulsion according to any one of the preceding claims, **characterized in that** it has a turbidity ranging from 60 to 400 NTU.

6. Nanoemulsion according to any one of the preceding claims, **characterized in that** the homopolymers and copolymers of ethylene oxide are chosen from:
(1) poly(ethylene oxides) having the following formula (I):
R-(CH₂-CH₂-O)ₙ-R' (I)
in which R is chosen from the hydroxyl, methoxy and amine groups, R' is a methyl group or a hydrogen, and n is a number ranging from 220 to 230,000;
(2) copolymers of ethylene oxide and of one or more oxyalkylenated monomers having the following formula (II) :
-(CHR-CHR'-O)- (II)
in which R and R', independently of each other, are hydrogen or an alkyl group comprising from 1 to 7 carbon atoms, at least one of R or R' being an alkyl group.

7. Nanoemulsion according to any one of the preceding claims, **characterized in that** the polyvinyl alcohols are compounds of the following formula (III): 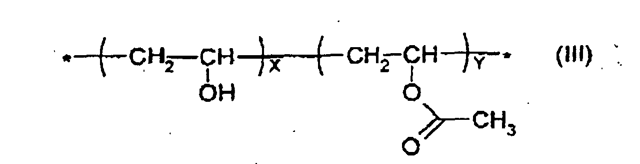 in which x is a mean number expressed as a percentage ranging from 70 to 100; y is a mean number equal to 100 - x.

8. Nanoemulsion according to any one of Claims 1 to 5, **characterized in that** the homopolymers and copolymers of vinylpyrrolidone are chosen from:
- polyvinylpyrrolidones having the following formula (IV): 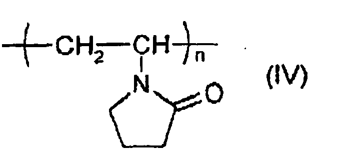
- copolymers of vinylpyrrolidone and of vinyl acetate;
- copolymers of vinylpyrrolidone and of vinylpyrrolidone derivatives with butene grafts;
- copolymers of vinylpyrrolidone and of maleic anhydride;
- copolymers of vinylpyrrolidone with polyvinyl alkyl ethers of the following formula (V) : 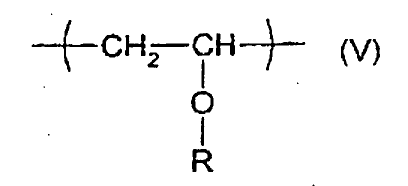 in which R is chosen from the alkyl groups containing from 1 to 7 carbon atoms;
- copolymers of vinylpyrrolidone and of N-vinyllactams;
- copolymers of vinylpyrrolidone with the neutral acrylic derivatives of the following formula (VI): 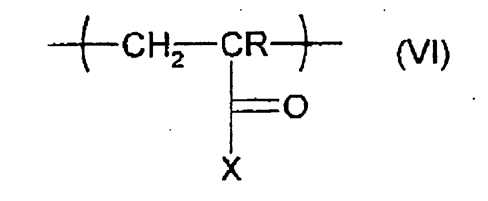 in which R is hydrogen or a methyl group, and X is chosen from the groups alkyl oxide of the type OR' where R' contains from 1 to 7 carbon atoms; hydroxylated and/or aminated alkyl oxide of the OR₁(OH)ₙ(NR₂R₃)ₘ type where n and m are numbers ranging from 0 to 10, R₁ is an alkyl group containing from 1 to 7 carbon atoms; R₂ and R₃ are independently hydrogen or an alkyl group such that the sum of the carbon atoms of R₂ and R₃ ranges from 1 to 7; primary, secondary or tertiary amine of the NR₂R₃ type where R₂ and R₃ have the meaning indicated above.

9. Nanoemulsion according to any one of the preceding claims, **characterized in that** the homopolymers and copolymers of vinylcaprolactam are chosen from:
- polyvinylcaprolactams which have the following formula (VII) : 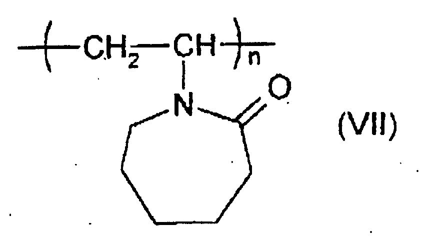
- copolymers of vinylcaprolactams obtained from vinylcaprolactam and from one or more of the following monomers:
- vinyl acetate;
- N-vinyllactam;
- maleic anhydride;
- vinyl alkyl ethers of formula (V);
- neutral acrylic derivatives of formula (VI).

10. Nanoemulsion according to any one of the preceding claims, **characterized in that** the copolymers of polyvinyl methyl ether are obtained from vinyl methyl ether and from one or more of the following monomers:
- vinyl alkyl ethers of formula (V) ;
- vinyl acetate;
- N-vinyllactam;
- maleic anhydride;
- neutral acrylic derivatives of formula (VI).

11. Nanoemulsion according to any one of the preceding claims, **characterized in that** the neutral acrylic copolymers and homopolymers are chosen from:
- neutral water-soluble acrylic polymers having the following formula (IX): 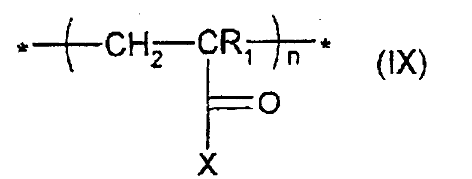 in which R₁ is hydrogen or a methyl group, and X is chosen from
(a) alkylamino groups of the NR₂R₃ type, such that the corresponding acrylic polymer is water-soluble, where R₂ and R₃ are independently hydrogen or an alkyl group such that the sum of the carbon atoms of R₂ and R₃ ranges from 1 to 7;
(b) hydroxylated and/or aminated. alkyl oxide groups of the OR₂(OH)ₙ(NR₃R₄)ₘ type where n and m are numbers ranging from 0 to 10, R₂ is an alkyl group containing from 1 to 7 carbon atoms; R₃ and R₄ are independently hydrogen or an alkyl group such that the sum of the carbon atoms of R₃ and R₄ ranges from 1 to 7, these groups being such that the corresponding acrylic derivative is water-soluble;
- copolymers of an acrylic derivative of formula (IX) and of one or more of the following neutral monomers:
- vinyl acetate;
- N-vinyllactam;
- maleic anhydride;
- vinyl alkyl ethers of formula (V);
- neutral acrylic derivative of formula (VI).

12. Nanoemulsion according to any one of the preceding claims, **characterized in that** the C₁-C₂ alkyl celluloses and their neutral derivatives are chosen from hydroxyethyl cellulose; ethylhydroxyethyl cellulose; methyl cellulose and methylhydroxyalkyl celluloses.

13. Nanoemulsion according to any one of the preceding claims, **characterized in that** the polymer is hydroxypropyl guar.

14. Nanoemulsion according to any one of the preceding claims, **characterized in that** the quantity of water-soluble nonionic polymer(s) ranges from 0.01 to 20% by weight relative to the total weight of the composition.

15. Nanoemulsion according to any one of the preceding claims, **characterized in that** the nonionic amphiphilic lipid is chosen from:
1) silicone surfactants,
2) amphiphilic lipids which are liquid at a temperature of less than or equal to 45°C chosen from esters of at least one polyol and of at least one fatty acid comprising at least one saturated or unsaturated and linear or branched C₈-C₂₂ alkyl chain,
3) esters of fatty acid and of sugar and ethers of fatty alcohol and of sugar,
4) surfactants which are solid at a temperature of less than or equal to 45°C chosen from glycerol fatty esters, sorbitan fatty esters and oxyethylenated sorbitan fatty esters, ethoxylated fatty ethers and ethoxylated fatty esters,
5) block copolymers of ethylene oxide and of propylene oxide,
and their mixtures.

16. Nanoemulsion according to any one of the preceding claims, **characterized in that** the amount of amphiphilic lipid ranges from 0.2 to 15% by weight with respect to the total weight of the composition.

17. Nanoemulsion according to any one of the preceding claims, **characterized in that** the additional ionic amphiphilic lipid is present in an amount ranging from 0.01 to 10% by weight with respect to the total weight of the composition.

18. Nanoemulsion according to any one of the preceding claims, **characterized in that** the amount of oily phase ranges from 2 to 40% by weight with respect to the total weight of the composition.

19. Nanoemulsion according to any one of the preceding claims, **characterized in that** it constitutes a cosmetic or dermatological composition.

20. Nanoemulsion according to any one of Claims 1 to 18, **characterized in that** it constitutes an ophthalmic vehicle.

21. Nanoemulsion according to any one of Claims 1 to 18, **characterized in that** it constitutes a pharmaceutical composition.

22. Cosmetic use of the nanoemulsion according to any one of Claims 1 to 18 or of the composition according to Claim 19 for caring for, treating and/or making up the skin, the face and/or the scalp.

23. Cosmetic use of the nanoemulsion according to any one of Claims 1 to 18 or of the composition according to Claim 19 for caring for and/or treating the hair.

24. Cosmetic process for caring for and/or moisturizing the skin, mucous membranes and/or scalp, **characterized in that** a nanoemulsion according to any one of Claims 1 to 18 or a composition according to Claim 19 is applied to the skin, mucous membranes and/or scalp.

25. Use of the nanoemulsion according to any one of Claims 1 to 18 in the manufacture of a composition intended for the treatment of dry skin.

26. Use of the nanoemulsion according to any one of Claims 1 to 18 in the manufacture of an ophthalmological composition.

27. Method for thickening an oil-in-water nanoemulsion having oil globules whose number-average size is less than 100 nm, consisting in adding to the said nanoemulsion at least one water-soluble nonionic polymer totally free of hydrophobia chain and chosen from homopolymers and copolymers of ethylene oxide having a molar mass equal to or greater than 10,000 g/mol; polyvinyl alcohols; homopolymers and copolymers of vinylpyrrolidone; homopolymers and copolymers of vinylcaprolactam; homopolymers and copolymers of polyvinyl methyl ether; neutral acrylic homopolymers and copolymers; C₁-C₂ alkyl celluloses and their derivatives; C₁-C₃ alkyl guar or C₁-C₃ hydroxyalkyl guar, the presence of the polymer increasing the viscosity of the nanoemulsion by at least a factor of 5 for a polymer concentration equal to 1% by weight.

## Patentansprüche

1. Öl-in-Wasser-Nanoemulsion, die eine in einer wässerigen Phase dispergierte Ölphase aufweist, wobei das Zahlenmittel der Größe der Ölkügeichen unter 100 nm liegt, **dadurch gekennzeichnet, dass** sie (i) mindestens ein amphiphiles Lipid, das unter nichtionischen amphiphilen Lipiden und anionischen amphiphilen Lipiden ausgewählt ist, (ii) mindestens ein wasserlösliches nichtionisches Polymer, das überhaupt keine hydrophobe Gruppe aufweist und das unter Homopolymeren und Copolymeren von Ethylenoxid mit einer Molmasse von größer oder gleich 10 000 g/mol, Polyvinylalkoholen, Homopolymeren und Copolymeren von Vinylpyrrolidon, Homopolymeren und Copolymeren von Vinylcaprolactam, Homopolymeren und Copolymeren von Polyvinylmethylether, neutralen Acrylhomopolymeren und Acrylcopolymeren, C₁₋₂-Alkylcellulosen und ihren Derivaten und C₁ ₃-Alkylguarverbindungen oder C₁₋₃-Hydroxyalkylguarverbindungen ausgewählt ist, und (iii) mindestens ein zusätzliches ionisches amphiphiles Lipid enthält, das unter:
- Alkalisalzen von Dicetylphosphat und Dimyristylphosphat,
- Alkalisalzen von Cholesterinsulfat,
- Alkalisalzen von Cholesterinphosphat,
- Lipoaminosäuren und ihren Salzen,
- Natriumsalzen von Phosphatidsäure,
- Phospholipiden,
- Alkylsulfonsäurederivaten,
- quartären Ammoniumsalzen,
- Fettaminen und
- den Gemischen dieser Verbindungen
ausgewählt ist, und dadurch, dass das Gewichtsverhältnis der Menge der Ölphase zu der Menge des amphiphilen Lipids im Bereich von 2 bis 10 liegt.

2. Nanoemulsion nach Anspruch 1, **dadurch gekennzeichnet, dass** das Gewichtsverhältnis der Menge der Ölphase zu der Menge des amphiphilen Lipids im Bereich von 2 bis 6 liegt.

3. Nanoemulsion nach einem der vorhergehenden Ansprüche, **dadurch gekennzeichnet, dass** das Zahlenmittel der Größe der Ölkügelchen im Bereich von 20 bis 80 nm liegt.

4. Nanoemulsion nach einem der vorhergehenden Ansprüche, **dadurch . gekennzeichnet, dass** die Viskosität der Nanoemulsion durch die Anwesenheit des Polymers um mindestens Faktor 5 erhöht wird.

5. Nanoemulsion nach einem der vorhergehenden Ansprüche, **dadurch gekennzeichnet, dass** sie einen Trübungswert von 60 bis 400 NTU aufweist.

6. Nanoemulsion nach einem der vorhergehenden Ansprüche, **dadurch gekennzeichnet, dass** die Homopolymere und Copolymere von Ethylenoxid ausgewählt sind unter:
(1) Poly-Ethylenoxiden der folgenden Formel (I):
R-(CH₂-CH₂-O)ₙ-R' (I),
worin:
- R unter den Gruppen Hydroxy, Methoxy und Amino ausgewählt ist,
- R' eine Methylgruppe oder Wasserstoff bedeutet und
- n eine Zahl von 220 bis 230 000 ist.
(2) Copolymeren von Ethylenoxid und einem oder mehreren alkoxylierten Monomeren der folgenden Formel (II):
-(CHR-CHR'-O)- (II),
worin R und R' unabhängig voneinander ein Wasserstoffatom oder eine Alkylgruppe mit 1 bis 7 Kohlenstoffatomen bedeuten, wobei mindestens eine der Gruppen R oder R' eine Alkylgruppe ist.

7. Nanoemulsion nach einem der vorhergehenden Ansprüche, **dadurch gekennzeichnet, dass** es sich bei den Polyvinylalkoholen um Verbindungen der folgenden Formel (III) handelt: 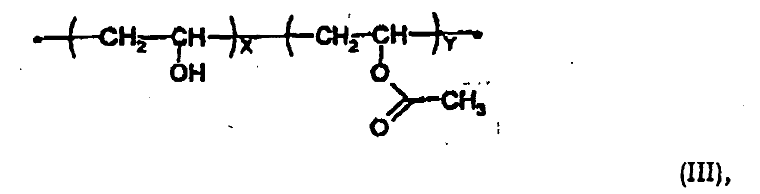 worin bedeuten:
- x einen in Prozent ausgedrückten Mittelwert im Bereich von 70 bis 100 und
- y einen Mittelwert, der 100 - x ist.

8. Nanoemulsion nach einem der Ansprüche 1 bis 5, **dadurch gekennzeichnet, dass** die Homopolymere und Copolymere von Vinylpyrrolidon ausgewählt sind unter:
- Polyvinylpyrrolidonen der folgenden Formel (IV): 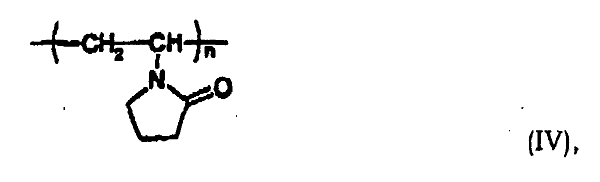
- Copolymeren von Vinylpyrrolidon und Vinylacetat,
- Copolymeren von Vinylpyrrolidon und Vinylpyrrolidonderivaten mit aufgepfropftem Buten,
- Copolymeren von Vinylpyrrolidon und Maleinsäureanhydrid,
- Copolymeren von Vinylpyrrolidon und Polyvinylalkylethern der folgenden Formel (V): 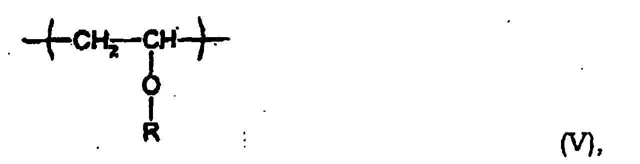
worin R unter Alkylgruppen mit 1 bis 7 Kohlenstoffatomen ausgewählt ist,
- Copolymeren von Vinylpyrrolidon und N-Vinyllactamen,
- Copolymeren von Vinylpyrrolidon und neutralen Acrylderivaten der folgenden Formel (VI): 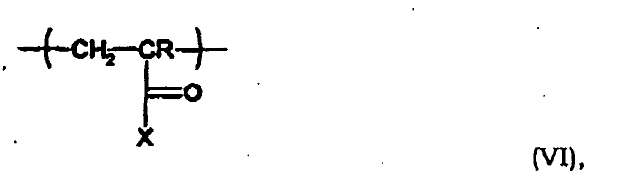
worin:
- R Wasserstoff oder eine Methylgruppe bedeutet und
- X ausgewählt ist unter Alkyloxidgruppen vom Typ OR', worin R' 1 bis 7 Kohlenstoffatome enthält, hydroxylierten und/oder aminierten Alkyloxidgruppen vom Typ OR₁(OH)ₙ(NR₂R₃)ₘ, worin n und m Zahlen von 0 bis 10 bedeuten, R₁ eine Alkylgruppe mit 1 bis 7 Kohlenstoffatomen ist und R₂ und R₃ unabhängig voneinander Wasserstoff oder eine Alkylgruppe bedeuten, die so ist, dass die Summe der Kohlenstoffatome von R₂ und R₃ im Bereich von 1 bis 7 liegt, und primären, sekundären oder tertiären Aminogruppen vom Typ NR₂R₃, worin R₂ und R₃ die oben angegebenen Bedeutungen aufweisen.

9. Nanoemulsion nach einem der vorhergehenden Ansprüche, **dadurch gekennzeichnet, dass** die Homopolymere und Copolymere von Vinylcaprolactam ausgewählt sind unter:
- Polyvinylcaprolactarnen, die der folgenden Formel (VII) entsprechen: 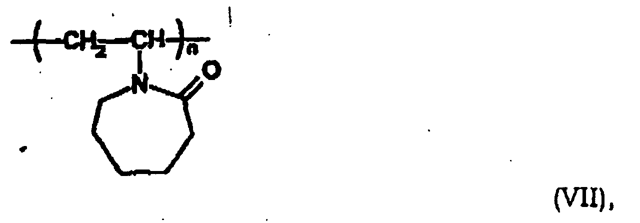
- Copolymeren von Vinylcaprolactamen, die aus Vinylcaprolactam und einem oder mehreren der folgenden Monomere erhalten werden:
- Vinylacetat,
- N-Vinyllactam,
- Maleinsäureanhydrid,
- Vinylalkylethern der Formel (V) und
- neutralen Acrylderivaten der Formel (VI).

10. Nanoemulsion nach einem der vorhergehenden Ansprüche, **dadurch gekennzeichnet, dass** die Copolymere von Polyvinylmethylether aus Vinylmethylether und einem oder mehreren der folgenden Monomere erhalten werden:
- Vinylalkylethern der Formel (V),
- Vinylacetat,
- N-Vinyllactam,
- Maleinsäureanhydrid und
- neutralen Acrylderivaten der Formel (VI).

11. Nanoemulsion nach einem der vorhergehenden Ansprüche, **dadurch gekennzeichnet, dass** die neutralen Acrylhomopolymere und Acrylcopolymere ausgewählt sind unter:
- neutralen wasserlöslichen Acrylpolymeren der folgenden Formel (IX): 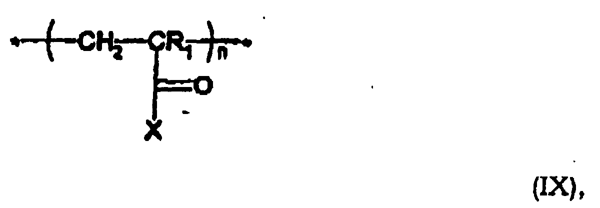
worin:
- R₁ ein Wasserstoffatom oder eine Methylgruppe bedeutet und
- X ausgewählt ist unter (a) alkylaminierten Gruppen vom Typ NR₂R₃, die so sind, dass das entsprechende Acrylpolymer wasserlöslich ist, wobei R₂ und R₃ unabhängig voneinander Wasserstoff oder eine Alkylgruppe bedeuten, die so ist, dass die Summe der Kohlenstoffatome von R₂ und R₃ im Bereich von 1 bis 7 liegt, oder (b) hydroxylierten und/oder aminierten Alkyloxidgruppen vom Typ OR₂(OH)ₙ(NR₃R₄)ₘ, worin n und m Zahlen von 0 bis 10 sind, R₂ eine Alkylgruppe mit 1 bis 7 Kohlenstoffatomen bedeutet und R₃ und R₄ unabhängig voneinander Wasserstoff oder eine Alkylgruppe bedeuten, die so ist, dass die Summe der Kohlenstoffatome von R₃ und R₄ im Bereich von 1 bis 7 liegt, wobei die Gruppen so sind, dass das entsprechende Acrylderivat wasserlöslich ist,
und
- Copolymeren eines Acrylderivats der Formel (IX) und eines oder mehrerer der folgenden neutralen Monomere:
- Vinylacetat,
- N-Vinyllactam,
- Maleinsäureanhydrid,
- Vinylalkylethern der Formel (V) und
- neutralen Acrylderivaten der Formel (VI).

12. Nanoemulsion nach einem der vorhergehenden Ansprüche, **dadurch gekennzeichnet, dass** die C₁₋₂-Alkylcellulosen und ihre neutralen Derivate unter Hydroxyethylcellulose, Ethylhydroxyethylcellulose, Methylcellulose und Methylhydroxyalkylcellulosen ausgewählt sind.

13. Nanoemulsion nach einem der vorhergehenden Ansprüche, **dadurch gekennzeichnet, dass** das Polymer Hydroxypropylguar ist.

14. Nanoemulsion nach einem der vorhergehenden Ansprüche, **dadurch gekennzeichnet, dass** der Mengenanteil des wasserlöslichen nichtionischen Polymers (der wasserlöslichen nichtionischen Polymere) im Bereich von 0,01 bis 20 Gew.-%, bezogen auf das Gesamtgewicht der Zusammensetzung, liegt.

15. Nanoemulsion nach einem der vorhergehenden Ansprüche, **dadurch gekennzeichnet, dass** das nichtionische amphiphile Lipid unter:
1/ siliconhaltigen grenzflächenaktiven Stoffen,
2/ amphiphilen Lipiden, die bei einer Temperatur von kleiner oder gleich 45 °C flüssig sind und die unter den Estern mindestens eines Polyols und mindestens einer Fettsäure mit mindestens einer C₈₋₂₂-Alkylgruppe, die gesättigt oder nicht gesättigt, geradkettig oder verzweigt ist, ausgewählt sind,
3/ Estern einer Fettsäure und eines Zuckers und Ethern eines Fettalkohols und eines Zuckers,
4/ grenzflächenaktiven Stoffen, die bei einer Temperatur von kleiner oder gleich 45 °C fest sind und die unter Glycerinfettsäureestern, Sorbitanfettsäureestern und ethoxylierten Sorbitanfettsäureestern, ethoxylierten Fettethern und ethoxylierten Fettestern ausgewählt sind,
5/ Blockcopolymeren von Ethylenoxid und Propylenoxid und deren Gemischen ausgewählt ist.

16. Nanoemulsion nach einem der vorhergehenden Ansprüche, **dadurch gekennzeichnet, dass** der Mengenanteil des amphiphilen Lipids im Bereich von 0,2 bis 15 Gew.-%, bezogen auf das Gesamtgewicht der Zusammensetzung, liegt.

17. Nanoemulsion nach einem der vorhergehenden Ansprüche, **dadurch gekennzeichnet, dass** das zusätzliche ionische amphiphile Lipid in einem Mengenanteil von 0,01 bis 10 Gew.-%, bezogen auf das Gesamtgewicht der Zusammensetzung, vorliegt.

18. Nanoemulsion nach einem der vorhergehenden Ansprüche, **dadurch gekennzeichnet, dass** der Mengenanteil der Ölphase im Bereich von 2 bis 40 Gew.-%, bezogen auf das Gesamtgewicht der Zusammensetzung, liegt.

19. Nanoemulsion nach einem der vorhergehenden Ansprüche, **dadurch gekennzeichnet, dass** sie eine kosmetische oder dermatologische Zusammensetzung darstellt.

20. Nanoemulsion nach einem der Ansprüche 1 bis 18, **dadurch gekennzeichnet, dass** sie ein ophthalmischer Träger ist.

21. Nanoemulsion nach einem der Ansprüche 1 bis 18, **dadurch gekennzeichnet, dass** sie eine pharmazeutische Zusammensetzung ist.

22. Kosmetische Verwendung der Nanoemulsion nach einem der Ansprüche 1 bis 18 oder der Zusammensetzung nach Anspruch 19 zur Pflege, zur Behandlung und/oder zum Schminken der Haut, des Gesichts und/oder der Kopfhaut.

23. Kosmetische Verwendung der Nanoemulsion nach einem der Ansprüche 1 bis 18 oder der Zusammensetzung nach Anspruch 19 zur Pflege und/oder zur Behandlung der Haare.

24. Kosmetisches Verfahren zur Pflege und/oder zur Hydratisierung der Haut, der Schleimhäute und/oder der Kopfhaut, **dadurch gekennzeichnet, dass** eine Nanoemulsion nach einem der Ansprüche 1 bis 18 oder eine Zusammensetzung nach Anspruch 19 auf die Haut, die Schleimhäute und/oder die Kopfhaut aufgebracht wird.

25. Verwendung der Nanoemulsion nach einem der Ansprüche 1 bis 18 zur Herstellung einer Zusammensetzung, die zur Behandlung von trockener Haut vorgesehen ist.

26. Verwendung der Nanoemulsion nach einem der Ansprüche 1 bis 18 zur Herstellung einer ophthalmologischen Zusammensetzung.

27. Verfahren zum Verdicken einer Öl-in-Wasser-Nanoemulsion, die Ölkügelchen mit einem Zahlenmittel der Partikelgröße unter 100 nm aufweist, wobei das Verfahren darin besteht, mindestens ein wasserlösliches nichtionisches Polymer zu der Nanoemulsion zu geben, das überhaupt keine hydrophobe Gruppe aufweist und das unter Homopolymeren und Copolymeren von Ethylenoxid mit einer Molmasse von größer oder gleich 10 000 g/mol, Polyvinylalkoholen, Homopolymeren und Copolymeren von Vinylpyrrolidon, Homopolymeren und Copolymeren von Vinylcaprolactam, Homopolymeren und Copolymeren von Polyvinylmethylether, neutralen Acrylhomopolymeren und Acrylcopolymeren, C₁₋₂-Alkylcellulosen und ihren Derivaten und C₁ ₃-Alkylguarverbindungen oder C₁₋₃-Hydroxyalkylguarverbindungen ausgewählt ist, wobei die Viskosität der Nanoemulsion durch die Anwesenheit des Polymers um mindestens Faktor 5 erhöht wird, wenn das Polymer in einer Konzentration von 1 Gew.-% vorliegt.
